# FASCICULE DE BREVET EUROPEEN

(11) **EP 2 897 474 B1**
(45) Date de publication et mention de la délivrance du brevet: **15.11.2017**
(21) Numéro de dépôt: 13779292.5
(22) Date de dépôt: 23.09.2013
(51) Int. Cl.: A23L 33/17, A23J 1/00, A23J 1/20, A23J 3/06, A23J 3/14

(54) **ASSEMBLAGE D'AU MOINS UNE PROTÉINE VÉGÉTALE ET D'AU MOINS UNE PROTÉINE LAITIÈRE**
ANORDNUNG AUS MINDESTENS EINEM PFLANZENPROTEIN UND MINDESTENS EINEM MILCHPROTEIN
ASSEMBLY OF AT LEAST ONE VEGETABLE PROTEIN AND AT LEAST ONE DAIRY PROTEIN

(30) Priorité: 21.09.2012 FR 1258903
(43) Date de publication de la demande: 29.07.2015
(73) Titulaire: Roquette Frères, 62136 Lestrem (FR); Ingredia, 62000 Arras (FR); Institut National de la Recherche Agronomique (INRA), 75007 Paris (FR)
(72) Inventeur: BOURSIER, Bernard, F-62138 Violaines (FR); MORETTI, Emmanuelle, F-59800 Lille (FR); RIBADEAU-DUMAS, Guillaume, F-59237 Verlinghem (FR); BELAID, Saliha, 80000 Amiens (FR); RIAUBLANC, Alain, F-44850 Ligne (FR); GUEGUEN, Jacques, F-44240 La Chapelle Sur Erdre (FR); LEPOUDERE, Anne, F-56380 Guer (FR); SNAPPE, Jean-Jacques, F-62149 Festubert (FR); COLIN, Isabelle, F-62223 Anzin Saint Aubin (FR)
(74) Mandataire: Cabinet Plasseraud
(86) Numéro de dépôt international: PCT/FR2013/052215
(87) Numéro de publication internationale: WO 2014/044990

(56) Documents cités:
- WO-A1-2008/052062
- WO-A1-2010/126353
- FR-A1- 2 497 634
- US-A- 3 873 751

## Description

### DOMAINE DE L'INVENTION

La présente invention a pour objet un procédé de production d'un assemblage d'au moins une protéine laitière et d'au moins une protéine végétale. Il a également pour objet l'assemblage susceptible d'être obtenu par ledit procédé, ainsi que ses utilisations, en particulier dans le domaine agroalimentaire.

### ARRIERE-PLAN TECHNOLOGIQUE

Avec les glucides et les lipides, les protéines constituent une partie importante de notre alimentation. Les protéines consommées proviennent généralement soit d'une origine animale (viandes, poissons, oeufs, produits laitiers...), soit d'une origine végétale (céréales, légumineuses...).
Leur rôle nutritionnel est de fournir des acides aminés et de l'énergie, substrats nécessaires à la synthèse des protéines de l'organisme.
Les protéines se composent d'un enchaînement d'acides aminés. Il existe 20 acides aminés dont 9 sont indispensables à l'homme, car l'organisme ne sait pas les synthétiser et ils doivent donc être apportés par l'alimentation.
Dans l'approche traditionnelle, la qualité des protéines est évaluée à partir de leur teneur en acides aminés indispensables. Il est notamment connu qu'en règle générale les protéines d'origine animale sont plus riches en certains acides aminés indispensables que les protéines végétales.
Les protéines de lait présentent un intérêt nutritionnel intéressant ; par contre leur coût est élevé et peut être un frein à leur utilisation. Les industriels cherchent donc des protéines de substitution ; et les protéines végétales sont des protéines de substitution attractives.

De nombreuses demandes de brevet décrivent déjà l'utilisation de protéines végétales pour remplacer tout ou partie des protéines d'origine animale dans des aliments. Toutefois, les protéines de substitution actuellement disponibles sur le marché ne possèdent pas nécessairement des propriétés fonctionnellement optimales et intéressantes, équivalentes aux propriétés fonctionnelles des ingrédients protéiques fonctionnelles d'origine animale..
Le document US 3 873 751 décrit un produit de substitution au lait à partir de lactosérum et de protéines végétales, notamment de soja.
Le document WO 2008/052062 décrit une composition de granules protéiques comprenant une composition de protéine végétale et de lactosérum ou alors une composition de protéine végétale et de produit laitier.
Le document WO 2010/126353 décrit une composition comprenant des protéines de pois et des protéines laitières, notamment du lactosérum et/ou des caséines. D'autres protéines peuvent être incorporées, par exemple des protéines de soja.
Le document FR 2 497 634 concerne un procédé d'obtention d'un produit protéique ressemblant au lait et ayant des meilleures caractéristiques de suspension dans l'eau. La composition décrite dans ce document comprend une matière protéique végétale comme le soja, et du lactosérum.

Les protéines jouent un rôle important sur la qualité organoleptique de nombreux aliments frais ou manufacturés, comme par exemple la consistance et la texture de la viande et produits à base de viande, du lait et dérivés, des pâtes et du pain. Ces qualités des aliments dépendent très fréquemment de la structure, des propriétés physico-chimiques et des propriétés fonctionnelles des composants protéiques des aliments.

Le terme « propriétés fonctionnelles » des ingrédients alimentaires signifie dans la présente demande toute propriété non nutritionnelle qui influence l'utilité d'un ingrédient dans un aliment. Ces diverses propriétés contribuent à l'obtention des caractéristiques finales désirées de l'aliment. Quelques unes de ces propriétés fonctionnelles sont la solubilité, l'hydratation, la viscosité, la coagulation, la stabilisation, la texturation, la formation de pâte, les propriétés moussantes, les capacités émulsifiantes et gélifiantes. Les protéines jouent également un rôle important dans les propriétés sensorielles des matrices alimentaires dans lesquelles elles sont utilisées, et il existe une réelle synergie entre les propriétés fonctionnelles et les propriétés sensorielles.

Les propriétés fonctionnelles des protéines ou fonctionnalité sont donc les propriétés physiques ou physico-chimiques qui ont une incidence sur les qualités sensorielles des systèmes alimentaires générées au cours des transformations technologiques, de la conservation ou des préparations culinaires domestiques.

On constate quelque soit l'origine de la protéine qu'elle intervient sur la couleur, la flaveur et/ou la texture d'un produit. Ces caractéristiques organoleptiques interviennent de façon déterminante dans le choix du consommateur et elles sont dans ce cas largement prises en compte par les industriels.

La fonctionnalité des protéines est le résultat d'interactions moléculaires de ces dernières avec leur environnement (autres molécules, pH, température...). Ces propriétés sont généralement classées en 3 groupes :
- propriétés d'hydratation qui regroupent les interactions de la protéine avec l'eau : cela recouvre les propriétés d'absorption, de rétention, de mouillabilité, de gonflement, d'adhérence, de dispersion, de viscosité....
- propriétés de structuration qui regroupent les propriétés d'interaction Protéine-Protéine : cela recouvre les phénomènes de précipitation, de coagulation, de gélification....
- propriétés de surface qui regroupent les propriétés d'interaction des protéines avec d'autres structures polaires ou apolaires en phase liquide ou gazeuse : cela recouvre les propriétés émulsifiantes, moussantes...

Ces différentes propriétés ne sont pas indépendantes les unes des autres car une propriété fonctionnelle peut résulter de plusieurs types d'interactions ou de plusieurs propriétés fonctionnelles.

Les Sociétés Demanderesses ont constaté qu'il existait un réel besoin, non satisfait, de disposer d'une composition possédant des propriétés fonctionnelles intéressantes, pouvant être employée dans l'alimentation comme un substitut au moins partiel des protéines d'origine animale.

Dans ce contexte, les Sociétés Demanderesses ont mis au point un procédé particulier permettant d'obtenir une nouvelle composition comprenant au moins une protéine laitière et au moins une protéine végétale possédant des propriétés fonctionnelles et/ou sensorielles améliorées.

### RESUME DE L'INVENTION

La présente invention a ainsi pour objet un procédé d'obtention d'un assemblage d'au moins une protéine végétale et d'au moins une protéine laitière selon la revendication 1, comprenant les étapes consistant à se munir d'une composition comprenant au moins une protéine végétale, à se munir d'une composition comprenant au moins une protéine laitière et à mélanger la composition comprenant au moins une protéine végétale et la composition comprenant au moins une protéine laitière, et en outre une ou plusieurs étapes, identiques ou différentes de traitement modifiant la conformation des protéines.

La présente invention concerne également un assemblage d'au moins une protéine laitière et d'au moins une protéine végétale susceptible d'être obtenu par le procédé décrit ci-dessus. Cet assemblage présente des propriétés fonctionnelles et/ou sensorielles améliorées par rapport aux propriétés fonctionnelles et/ou sensorielles qui seraient obtenues par la simple juxtaposition de ces protéines, par exemple dans le mélange à sec des deux types de protéines. L'assemblage d'au moins une protéine végétale et d'au moins une protéine laitière selon la présente invention permet ainsi d'obtenir une réelle synergie au niveau des propriétés finales obtenues. Cela signifie que les propriétés de chacune des protéines utilisées ne sont pas juste ajoutées et cumulées, mais qu'elles sont soit améliorées soit nouvelles. Cette synergie est démontrée notamment dans les exemples ci-après.

La présente invention concerne enfin l'utilisation dudit assemblage dans différents secteurs industriels, et plus particulièrement dans le domaine agroalimentaire. L'assemblage peut être utilisé en tant qu'agent fonctionnel, et de préférence pour sa solubilité ou en tant qu'agent émulsifiant, moussant, gélifiant, viscosant, foisonnant, rétenteur d'eau et/ou pouvant réagir au traitement thermique.

### DESCRIPTION DE LA FIGURE

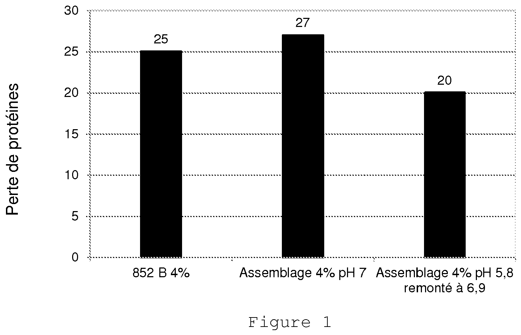

La figure 1 présente les pertes de protéines mesurées dans les sérums de différents assemblages (PROMILK 852 B + Floculat) de la présente invention ainsi que les pertes de protéines obtenues pour la protéine laitière (PROMILK 852 B 4%) seule lors de coagulation à la présure.

### DESCRIPTION DETAILLEE DES MODES DE REALISATION

Communément, on appelle assemblage de protéines la réunion de plusieurs protéines formant ensemble une structure tridimensionnelle particulière.

En effet, les protéines sont formées d'une succession d'acides aminés. La partie radicale des acides aminés porte des fonctions chimiques différentes. Ainsi, il peut y avoir des interactions entre les radicaux des acides aminés, typiquement des interactions hydrophobes, des liaisons hydrogènes, des liaisons ioniques et des ponts disulfures. Les interactions entre radicaux ont pour effet de provoquer le repliement des protéines sur elles-mêmes et entre elles pour adopter une structure supramoléculaire tridimensionnelle. En cela, l'assemblage de protéines se distingue du simple mélange : les protéines ne sont pas simplement mélangées physiquement, mais forment ensemble une nouvelle structure, ayant par exemple une taille, une morphologie et une composition particulière.

Le procédé objet de la présente invention comprend les étapes consistant à se munir d'une composition comprenant au moins une protéine végétale, à se munir d'une composition comprenant au moins une protéine laitière et à mélanger la composition comprenant au moins une protéine végétale et la composition comprenant au moins une protéine laitière.

Dans la présente invention, le terme « protéine végétale » désigne toutes les protéines issues des céréales, des oléagineux, des légumineuses et des tubercules, ainsi que toutes les protéines issues des algues et des microalgues, utilisée seule ou en mélange, choisie dans la même famille ou dans des familles différentes.

Ces protéines végétales peuvent être utilisées seules ou en mélanges, choisies dans la même famille ou dans des familles différentes.

Par « algues » et « microalgues », on entend dans la présente demande des organismes eucaryotes dépourvus de racines, de tige et de feuille, mais possédant de la chlorophylle ainsi que d'autres pigments accessoires à la photosynthèse productrice d'oxygène. Elles sont bleues, rouges, jaunes, doré et brun ou encore vertes. Elles représentent plus de 90 % des végétaux marins et 18 % du règne végétal, avec leurs 40 000 à 45 000 espèces. Les algues sont des organismes extrêmement variés tant par leur taille et leur forme que par leur structure cellulaire. Elles vivent en milieu aquatique ou très humide. Elles contiennent de nombreuses vitamines et oligo-éléments, et sont de véritables concentrés d'actifs stimulants et bienfaisants pour la santé et la beauté. Elles ont des vertus anti-inflammatoires, hydratantes, adoucissantes, régénérantes, raffermissantes, anti-âge. Elles possèdent également des caractéristiques « technologiques » qui permettent d'apporter de la texture à un produit alimentaire. En effet, les additifs E400 à E407 sont des composés extraits d'algues, dont on utilise les propriétés épaississantes, gélifiantes, émulsifiantes et stabilisantes.

Les microalgues au sens strict sont des algues microscopiques. Unicellulaires ou pluricellulaires indifférenciées, ce sont des micro-organismes photosynthétiques séparés en deux groupes polyphylétiques : les eucaryotes et les procaryotes. Vivant dans les milieux fortement aqueux, elles peuvent posséder une mobilité flagellaire.

Selon un mode préférentiel, les microalgues sont choisies parmi le groupe constitué par *Chlorella, Spirulina* et *Odontella.*

Selon un mode encore plus préférentiel, les microalgues de la présente invention sont issues du genre *Chlorella,* et de préférence de *Chlorella vulgaris, Chlorella pyrenoidosa, Chlorella regularis, Chlorella sorokiniana,* et de façon encore plus préférentielle de *Chlorella vulgaris.*

Par « céréales », on entend dans la présente demande des plantes cultivées de la famille des graminées produisant des grains comestibles, comme par exemple le blé, l'avoine, le seigle, l'orge, le maïs, le sorgho, le riz. Les céréales sont souvent moulues sous forme de farine, mais se présentent aussi sous forme de grains et parfois sous forme de plante entière (fourrages).

Par « tubercules », on entend dans la présente demande tous les organes de réserve, généralement souterrains, qui assurent la survie des plantes pendant la saison d'hiver et souvent leur multiplication par voie végétative. Ces organes sont renflés par l'accumulation de substances de réserve. Les organes transformés en tubercules peuvent être :
- la racine : carotte, panais, manioc, konjac,
- le rhizome : pomme de terre, topinambour, crosne du Japon, patate douce,
- la base de la tige (plus précisément l'hypocotyle) : chou-rave, cèleri-rave,
- l'ensemble racine et hypocotyle : betterave, radis.

Par « oléagineux », on désigne dans la présente demande les plantes cultivées spécifiquement pour leurs graines ou leurs fruits riches en matières grasses, dont on extrait de l'huile à usage alimentaire, énergétique ou industriel, comme par exemple le colza, l'arachide, le tournesol, le soja, le sésame, le ricin.

Par « légumineuses », on entend au sens de la présente invention toutes plantes appartenant aux familles des césalpiniacées, des mimosacées ou des papilionacées et notamment toutes plantes appartenant à la famille des papilionacées comme, par exemple, le pois, le haricot, la fève, la fèverole, la lentille, la luzerne, le trèfle ou le lupin.

Cette définition inclut notamment toutes les plantes décrites dans l'un quelconque des tableaux contenus dans l'article de R. HOOVER et al., 1991 (HOOVER R. (1991) « Composition, structure, functionality and chemical modification of legume starches : a review » Can. J. Physiol. Pharmacol., 69 pp. 79-92).

Selon la présente invention, la protéine végétale appartient aux protéines de légumineuses.

En outre, la protéine de légumineuse est choisie dans le groupe consistant en la luzerne, le trèfle, le lupin, le pois, le haricot, la fève, la fèverole et la lentille, et leurs mélanges.

De manière plus préférée, ladite protéine de légumineuse est choisie dans le groupe consistant en le pois, le haricot, la fève et la fèverole, et leurs mélanges.

De manière encore plus préférée, ladite protéine de légumineuse est issue du pois.

Le terme « pois » étant ici considéré dans son acception la plus large et incluant en particulier :
- toutes les variétés de « pois lisse » (« *smooth pea »)* et « de pois ridés » (« *wrinkled pea* »), et
- toutes les variétés mutantes de « pois lisse » et de « pois ridé » et ce, quelles que soient les utilisations auxquelles on destine généralement lesdites variétés (alimentation humaine, nutrition animale et/ou autres utilisations).

Le terme « pois » dans la présente demande inclut les variétés de pois appartenant au genre *Pisum* et plus particulièrement aux espèces *sativum* et *aestivum.*

Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

De manière encore plus préférentielle, ladite protéine de légumineuse est issue du pois lisse.

En effet, le pois est la légumineuse à graines riches en protéines qui, depuis les années 70, s'est le plus développée en Europe et principalement en France, non seulement comme source protéique pour l'alimentation animale, mais aussi pour l'alimentation humaine.

Les protéines de pois sont constituées, comme toutes les protéines de légumineuses, de trois classes de protéines principales : les globulines, les albumines et les protéines dites insolubles.

L'intérêt des protéines de pois réside dans leurs bonnes capacités émulsifiantes, leur absence d'allergènicité, et leur faible coût, ce qui en fait un ingrédient fonctionnel économique.

De plus, les protéines de pois participent favorablement au développement durable et leur impact carbone est très positif. En effet, la culture du pois est respectueuse de l'environnement, et ne nécessite pas d'engrais azotés, car le pois fixe l'azote de l'air.

Selon la présente invention, la composition comprenant au moins une protéine végétale est de préférence une composition comprenant au moins une protéine de pois.

La composition comprenant au moins une protéine végétale, en particulier une protéine de pois, peut se présenter sous forme de solution, de dispersion ou de suspension ou sous forme solide, en particulier sous forme de poudre.

La composition comprenant au moins une protéine végétale, en particulier une protéine de pois, utilisée selon l'invention peut présenter avantageusement une teneur en protéines totales (N x 6,25), d'au moins 60 % en poids de produit sec. De préférence, on utilise dans le cadre de la présente invention une composition ayant une teneur en protéines élevée, comprise entre 70 % et 97 % en poids de produit sec, de préférence entre 76 % et 95 %, plus préférentiellement encore comprise entre 78 % et 88 %, et en particulier comprise entre 78 % en 85 %. La teneur en protéines totales est mesurée en effectuant le dosage de la fraction azotée soluble contenue dans l'échantillon selon la méthode de Kjeldahl. Puis, le taux de protéines totales est obtenu en multipliant le taux d'azote exprimé en pourcentage de poids de produit sec par le facteur 6,25.

En outre, ladite composition comprenant au moins une protéine végétale, en particulier une protéine de pois, peut présenter une teneur en protéines solubles, exprimée selon un test décrit ci-après de mesure de la solubilité dans l'eau des protéines, comprise entre 20 % et 99 %. De préférence, on utilise dans le cadre de la présente invention une composition ayant un taux élevé de protéines solubles compris entre 45 % et 90 %, plus préférentiellement encore entre 50 % et 80 %, et en particulier entre 55 % et 75 %.

Pour déterminer le taux de protéines solubles, on mesure la teneur en protéines solubles dans l'eau dont le pH est ajusté à 7,5 +/- 0,1 à l'aide d'une solution de HCl ou NaOH, par une méthode de dispersion d'une prise d'essai de l'échantillon dans de l'eau distillée, centrifugation et analyse du surnageant. Dans un bécher de 400 ml, on introduit 200,0 g d'eau distillée à 20°C +/- 2°C, et on place le tout sous agitation magnétique (barreau aimanté et rotation à 200 rpm). On ajoute exactement 5 g de l'échantillon à analyser. On agite pendant 30 min, et on centrifuge pendant 15 min à 4.000 rpm. On réalise, sur le surnageant la méthode de détermination de l'azote selon la méthode précédemment décrite.

Ces compositions comprenant au moins une protéine végétale, en particulier une protéine de pois, présentent de manière préférée plus de 50 %, plus préférentiellement plus de 60 %, encore plus préférentiellement plus de 70 %, encore plus préférentiellement plus de 80 %, et en particulier plus de 90 % de protéines de plus de 1 000 Da. La détermination du poids moléculaire de la protéine peut être réalisée selon la méthode décrite ci-après. En outre, ces compositions comprenant au moins une protéine végétale, en particulier une protéine de pois, présentent de manière préférée un profil de distribution des poids moléculaires constitué de :
- 1 % à 8 %, de préférence de 1,5 % à 4 %, et plus préférentiellement encore de 1,5 % à 3 %, de protéines de plus de 100 000 Da,
- 20 % à 55 %, de préférence de 25 % à 55 %, de protéines de plus de 15 000 Da et d'au plus 100 000 Da,
- 15 % à 30 % de protéines de plus de 5 000 Da et d'au plus 15 000 Da,
- et de 25 % à 55 %, de préférence de 25 % à 50 %, et plus préférentiellement encore de 25 % à 45 % de protéines d'au plus 5 000 Da.

Des exemples de compositions comprenant au moins une protéine végétale, en particulier une protéine de pois, selon l'invention, ainsi que le détail de la méthode de détermination des poids moléculaires peuvent être trouvés dans le brevet WO 2007/017572.

Selon la présente invention, la composition comprenant au moins une protéine végétale, en particulier une protéine de pois, peut être choisie dans le groupe constitué de concentrat de protéines végétales et d'isolat de protéines végétales, de préférence de concentrat de protéines de pois et d'isolat de protéines de pois. Les concentrats et les isolats de protéines végétales, et en particulier de pois, sont définis en regard de leur teneur en protéines (cf. la revue de J. GUEGUEN de 1983 dans Proceedings of european congress on plant proteins for human food (3-4) pp 267 - 304) :
- les concentrats de protéines végétales, et en particulier de pois, sont décrits comme présentant une teneur en protéines totales de 60 % à 75 % sur sec, et
- les isolats de protéines végétales, et en particulier de pois, sont décrits comme présentant une teneur en protéines totales de 90 % à 95 % sur sec,
les teneurs en protéines étant mesurées par la méthode de Kjeldhal, la teneur en azote étant multipliée par le facteur 6,25.

Dans un autre mode de réalisation de la présente invention, la composition comprenant au moins une protéine végétale, en particulier une protéine de pois, peut également être un « hydrolysat de protéines végétales », de préférence « hydrolysat de protéines de pois ». Les hydrolysats de protéines végétales, et en particulier de pois, sont définis comme des préparations obtenues par hydrolyse par voie enzymatique, par voie chimique, ou par les deux voies simultanément ou successivement, de protéines végétales, et en particulier de pois. Les hydrolysats de protéines comprennent une proportion plus élevée en peptides de différentes tailles et en acides aminés libres que la composition originale. Cette hydrolyse peut avoir un impact sur la solubilité des protéines. L'hydrolyse enzymatique et/ou chimique est par exemple décrite dans la demande de brevet WO 2008/001183. De préférence, l'hydrolyse de protéines n'est pas complète, c'est-à-dire ne résulte pas en une composition comprenant uniquement ou essentiellement des acides aminés et des petits peptides (de 2 à 4 acides aminés). Les hydrolysats préférés comprennent plus de 50 %, plus préférentiellement plus de 60 %, encore plus préférentiellement plus de 70 %, encore plus préférentiellement plus de 80 %, et en particulier plus de 90 % de protéines et de polypeptides de plus de 500 Da.

Les procédés de préparation d'hydrolysats de protéines sont bien connus de l'homme du métier et peuvent par exemple comprendre les étapes suivantes : dispersion des protéines dans l'eau pour obtenir une suspension, hydrolyse de cette suspension par le traitement choisi. Le plus souvent, il s'agira d'un traitement enzymatique combinant un mélange de différentes protéases, éventuellement suivi d'un traitement thermique destiné à inactiver les enzymes encore actives. La solution obtenue peut ensuite être filtrée sur une ou plusieurs membranes de façon à séparer les composés insolubles, éventuellement l'enzyme résiduelle et les peptides de haut poids moléculaire (supérieur à 10 000 daltons).

Dans un mode de réalisation préféré, la composition comprenant au moins une protéine végétale utilisée pour obtenir l'assemblage selon l'invention ne contient pas de gluten. Ce mode de réalisation est avantageux puisqu'il existe un certain nombre de personnes présentant une intolérance au gluten.

Le gluten est un groupe de protéines présentes dans les céréales, particulièrement dans le blé, mais également dans le seigle, l'orge, et l'avoine. Le gluten est, pour la plupart des personnes, une protéine normale qui est facilement digérée par l'intermédiaire de l'estomac. Cependant, une petite partie de la population est incapable de digérer le gluten. Ces personnes intolérantes au gluten sont désignées le plus généralement comme souffrant de la maladie coeliaque (aussi appelée psilosis coeliaque, entéropathie intolérante au gluten, ou entéropathie sensible au gluten). Cette maladie apparaît lorsqu'il y a une réaction chronique contre certaines chaînes protéiques présentes dans certaines céréales. Cette réaction engendre la destruction des villosités intestinales de l'intestin grêle, ce qui cause la malabsorption des nutriments et d'autres troubles plus ou moins graves. C'est une maladie très contraignante contre laquelle il n'existe à ce jour aucun traitement curatif.

Selon un mode optionnel de réalisation de l'invention, les compositions comprenant au moins une protéine végétale, en particulier une protéine de pois, peuvent subir un traitement thermique à température élevée et pendant un temps court, ledit traitement pouvant être choisi parmi les traitements HTST (High Temperature Short Time) et UHT (Ultra Haute Température). Ce traitement permet avantageusement de réduire les risques bactériologiques.

Dans la présente invention, le terme « protéine laitière » désigne toutes les protéines issues du lait et des produits dérivés du lait.

D'un point de vue chimique, les protéines du lait se distinguent en deux groupes : les caséines et les protéines sériques. Les caséines représentent 80 % des protéines totales du lait. Les protéines sériques, qui représentent les 20 % restants, sont solubles à pH 4,6. Parmi les protéines sériques, on trouve principalement le β-lactoglobuline, l'a-lactalbumine, la sérum albumine bovine, les immunoglobulines et la lactoferrine.

Selon un mode de réalisation de la présente invention, la composition comprenant au moins une protéine laitière peut être une composition comprenant au moins un rétentat de protéines de lait.

Selon un autre mode de réalisation de la présente invention, la composition comprenant au moins une protéine laitière peut être une composition comprenant au moins une caséine.

Selon un autre mode de réalisation de la présente invention, la composition comprenant au moins une protéine laitière peut être une composition comprenant au moins une protéine sérique.

Selon un autre mode de réalisation de la présente invention, la composition comprenant au moins une protéine laitière peut être une composition comprenant au moins une caséine et une protéine sérique.

La composition comprenant au moins une protéine laitière peut se présenter sous forme liquide ou sous forme solide, en particulier sous forme de poudre.

La composition comprenant au moins une protéine laitière peut en particulier être du lait ou un produit laitier.

Au niveau légal, il n'existe qu'une définition claire datant de 1909 définissant le lait d'origine animale : « Le lait est le produit intégral de la traite totale et ininterrompue d'une femelle laitière bien portante, bien nourrie et non surmenée. Il doit être recueilli proprement et ne pas contenir de colostrum. »

La dénomination « lait » sans indication de l'espèce animale de provenance est, au niveau de la législation française, réservée au lait de vache. Tout lait provenant d'une femelle laitière autre que la vache doit être désigné par la dénomination « lait » suivie de l'indication de l'espèce animale dont il provient, par exemple « lait de chèvre », « lait de brebis », « lait d'ânesse », « lait de bufflonne », etc. Toutefois, au sens de la présente invention, le lait et les produits laitiers peuvent avoir pour origine n'importe quelle espèce animale.
Par « produit laitier », on entend au sens de la présente invention tout produit obtenu à la suite d'un traitement quelconque du lait, qui peut contenir des additifs alimentaires et autres ingrédients fonctionnellement nécessaires au traitement (définition du CODEX Alimentarius).

Il est connu de procéder à la déshydratation du lait liquide pour obtenir de la poudre. La composition comprenant au moins une protéine laitière peut également être du lait en poudre, quelle que soit l'origine animale et le type du lait.

La composition comprenant au moins une protéine laitière peut en particulier être choisie dans le groupe constitué par les lactosérums et/ou, les babeurres, et/ou les perméats de lait ou de lactosérums et/ou les rétentats.

Le lactosérum, également appelé sérum ou « petit-lait », est la partie liquide issue de la coagulation du lait. On distingue deux sortes de lactosérums : ceux issus des fabrications acides de caséines ou de fromages à pâte fraîche (lactosérums acides), et ceux issus des fabrications de caséines présure et des fromages à pâte pressée cuite ou demi-cuite (lactosérums doux). Le lactosérum est généralement commercialisé sous forme de poudre. En dehors de l'eau, le lactosérum contient du lactose (de 70 % à 75 %), des protéines solubles (de 10 % à 13 %), des vitamines (thiamine-B1, riboflavine-B2 et pyridoxine-B6) et des minéraux (essentiellement du calcium).

Le babeurre, ou « lait battu », est traditionnellement issu du lait frais ou fermenté après la fabrication du beurre par barattage. Il est aussi fabriqué directement à partir du lait frais par ajout de ferments. Le babeurre peut être sous forme liquide, concentré ou en poudre.

En outre, les protéines laitières peuvent être extraites du lait ou des produits laitiers par des procédés bien connus de l'homme du métier. Ces protéines extraites peuvent être disponibles dans le commerce sous différentes formes, par exemple sous forme de poudre ou liquide, à différentes concentrations.

La composition comprenant au moins une protéine laitière peut être choisie dans le groupe constitué par :
- les compositions de protéines pouvant être obtenues par filtration du lait, en particulier des concentrats ou des isolats de protéines;
- les coprécipités de protéines pouvant être obtenus par traitement thermique et coprécipitation des protéines sériques avec les caséines ;
- les compositions de protéines sériques, en particulier des concentrats de protéines sériques ou des isolats de protéines sériques ;
- les caséines et les caséinates, en particulier les caséines natives, les caséines acides, les caséines présure, les caséinates de sodium, les caséinates de potassium et les caséinates de calcium ;
- les hydrolysats des protéines précités ;
utilisés seuls ou en combinaison avec d'autres produits laitiers comme le lactosérum déminéralisé, les perméats de lait ou de lactosérums.

Dans un premier mode de réalisation particulier, la composition comprenant au moins une protéine végétale est une composition comprenant une protéine végétale appartenant aux protéines des légumineuses, la protéine de légumineuse étant de préférence choisie dans le groupe consistant en la luzerne, le trèfle, le lupin, le pois, le haricot, la fève, la fèverole et la lentille, et leurs mélanges, et la composition comprenant au moins une protéine laitière est une composition comprenant au moins un rétentat de protéines de lait. Selon ce mode de réalisation particulier, la concentration protéique de l'assemblage est comprise entre 70% et 90% en poids sec, et plus particulièrement entre 78% et 85% en poids sec.

Dans un second mode de réalisation particulier, la composition comprenant au moins une protéine végétale est une composition comprenant une protéine végétale appartenant aux protéines des légumineuses, la protéine de légumineuse étant de préférence choisie dans le groupe consistant en la luzerne, le trèfle, le lupin, le pois, le haricot, la fève, la fèverole et la lentille, et leurs mélanges, et la composition comprenant au moins une protéine laitière est une composition comprenant au moins une caséine.

De préférence, la composition comprenant au moins une protéine végétale est une composition comprenant une protéine de pois, notamment une protéine de pois lisse et la composition comprenant au moins une protéine laitière est une composition comprenant au moins une caséine, notamment un rétentat de caséines micellaires.

Selon un mode de réalisation préféré, la composition comprenant au moins une protéine laitière est un concentrat de protéines totales ou un isolat de protéines totales.

Selon un autre mode de réalisation préféré, la composition comprenant au moins une protéine laitière est choisie dans le groupe constitué par les caséines natives, les caséines acides, les caséines présure, les caséinates de sodium, les caséinates de potassium et les caséinates de calcium.

Les concentrats de protéines laitières sont décrits comme présentant un contenu total en protéines supérieur à la matière d'origine.

Les isolats de protéines laitières sont décrits comme présentant un contenu en protéine totale supérieure à la matière d'origine et d'au moins 85 % sur sec. Dans les définitions précédentes, les teneurs en protéines sont mesurées par la méthode de Kjeldhal, la teneur en azote étant multipliée par le facteur 6,38 (facteur de conversion utilisé pour les protéines laitières).

Les protéines du lactosérum sont généralement obtenues par les procédés d'ultrafiltration, de concentration et de séchage.

Les caséines sont obtenues à partir de lait écrémé et sont précipitées soit par acidification au moyen d'acide ou de cultures bactériennes inoffensives et appropriées à l'alimentation humaine (caséines acides), soit par addition de présure ou d'autres enzymes coagulant le lait (caséines présure). Les caséinates sont les produits obtenus par séchage de caséines acides traitées avec des agents neutralisants. Selon les agents neutralisants utilisés, on obtient des caséinates de sodium, de potassium, de calcium et mixte (=co-neutralisation). Les caséines natives peuvent être obtenues à partir de lait écrémé par microfiltration tangentielle et diafiltration à l'eau.

Les hydrolysats de protéines laitières sont définis comme des préparations obtenues par hydrolyse par voie enzymatique, par voie chimique, ou par les deux voies simultanément ou successivement, de protéines laitières.

Les compositions comprenant au moins une protéine laitière extraite du lait ou des produits laitiers par des procédés bien connus de l'homme du métier présentent des teneurs en protéines totales différentes.

Lorsque les compositions sont sous la forme de poudre, les teneurs en protéines sont souvent exprimées en pourcentage, c'est-à-dire en poids de protéines sur le poids de poudre ; on parle alors de pourcentage massique.

Ainsi, il est connu qu'un lait en poudre contient environ 34% en poids de protéines. C'est-à-dire que dans 100g de poudre de lait il y a 34g de protéines.

On sait également qu'un lactosérum en poudre contient entre 10% et 15% en poids de protéines, et plus précisément aux alentours de 13% en poids.

Dans le cas des isoltas de protéines, le pourcentage de protéines de la poudre peut aussi être exprimé en pourcentage sur matière sèche. Ainsi un isolat de lait en poudre contenant 85% de protéines sur matière sèche est le même isolat que celui qui contient 80,75% en poids de protéine si la poudre contient 5% d'humidité (85% x95 / 100).

Selon un mode optionnel de réalisation de l'invention, les compositions comprenant au moins une protéine laitière précédemment décrites peuvent également subir un traitement thermique. Le traitement des aliments par la chaleur (ou traitement thermique) est aujourd'hui la plus importante technique de conservation de longue durée. Il a pour objectif de détruire ou d'inhiber totalement ou partiellement les enzymes et les microorganismes, dont la présence ou la prolifération pourrait altérer la denrée considérée ou la rendre impropre à la consommation.

L'effet d'un traitement thermique est lié au couple temps/température. De manière générale, plus la température est élevée et plus la durée est longue, plus l'effet sera important. Selon l'effet recherché, on distingue plusieurs traitements thermiques.

La stérilisation par la chaleur consiste à exposer les aliments à une température, généralement supérieure à 100°C, pendant une durée suffisante pour inhiber les enzymes et toute forme de microorganismes, même les bactéries sporulantes. Lorsque la stérilisation est réalisée à haute température (135°C à 150°C) pendant une durée n'excédant pas 15 secondes, on parle de stérilisation UHT (Ultra Haute Température). Cette technique a l'avantage de préserver la qualité nutritionnelle et organoleptique du produit stérilisé.

La pasteurisation est un traitement thermique modéré et suffisant permettant la destruction des microorganismes pathogènes et d'un grand nombre de microorganismes d'altération. La température du traitement est généralement inférieure à 100°C et la durée est de quelques secondes à quelques minutes. Lorsque la pasteurisation est réalisée à 72°C minimum pendant 15 secondes, on parle de pasteurisation HTST (High Temperature Short Time). La pasteurisation détruit les germes pathogènes et l'essentiel de la flore saprophyte. Mais tous les microorganismes n'étant pas éliminés par la pasteurisation, ce traitement thermique doit être suivi d'un brusque refroidissement. Les aliments pasteurisés sont alors habituellement conservés au froid (+4°C) afin de ralentir le développement des germes encore présents et la durée de conservation est limitée le plus souvent à une semaine.

La thermisation est un traitement thermique consistant à porter la solution à une température supérieure à 40°C et inférieure à 72°C. C'est une forme amoindrie de la pasteurisation. Son objectif principal est la réduction de la flore totale du lait, sans modifier pour autant ses caractéristiques technologiques.

Selon la présente invention, ledit traitement thermique pourra être choisi parmi les traitements pré-listés ci-dessus.

Le mélange de la composition comprenant au moins une protéine végétale et de la composition comprenant au moins une protéine laitière peut être réalisé selon les méthodes connues de l'homme du métier.

Selon un premier mode de réalisation, la composition comprenant au moins une protéine végétale et la composition comprenant au moins une protéine laitière sont sous forme liquide, le solvant étant de préférence l'eau. Le mélange peut éventuellement être dilué ou concentré.

Selon un deuxième mode de réalisation, l'une des deux compositions est sous forme liquide, le solvant étant de préférence l'eau, et l'autre est sous forme de poudre. Le mélange peut consister à introduire la composition en poudre dans la composition liquide.

Selon un troisième mode de réalisation, les deux compositions sont sous forme de poudre. Le mélange peut consister à mélanger les poudres sous forme sèche, puis éventuellement à les introduire dans de l'eau, ou à introduire l'une ou l'autre ou les deux compositions sous forme de poudre dans de l'eau, puis à les mélanger.

Le mélange des deux compositions peut être avantageusement suivi d'une agitation, de façon à homogénéiser le mélange. Il peut s'agir par exemple d'une agitation mécanique ou magnétique. Cette agitation peut être réalisée à une température comprise entre 1°C et 100°C, plus préférentiellement entre 2°C et 40°C, et encore plus préférentiellement entre 4°C et 35°C.

De préférence, le rapport (poids de matière azotée apportée par la composition comprenant au moins une protéine végétale) sur (poids de matière azotée apportée par la composition comprenant au moins une protéine laitière) est compris entre 99 :1 et 1 :99, plus préférentiellement entre 80 :20 et 20 :80, plus préférentiellement encore entre 63 :35 et 35 :65.

Dans le rapport précédent, les poids respectifs en protéines totales sont mesurés par la méthode en effectuant le dosage de la fraction azotée soluble contenue dans l'échantillon selon la méthode de Kjeldahl. Puis, le taux de protéines totales est obtenu en multipliant le taux d'azote exprimé en pourcentage de poids de produit sec par le facteur 6,25. Cette méthode est bien connue de l'homme du métier.

De préférence, la composition aqueuse obtenue après mélange a une teneur en protéines totales comprise entre 20 % et 100 % en poids de produit sec, plus préférentiellement entre 30 % et 100 %, et plus préférentiellement encore comprise entre 40 % et 100 %.

Selon un premier préférentiel de la présente invention, le mélange obtenu et contenant la composition comprenant au moins une protéine végétale et la composition comprenant au moins une protéine laitière subit une phase de repos, pendant laquelle aucun traitement n'est appliqué. Cette phase de repos peut durer de quelques minutes à plusieurs heures. Lorsque la durée n'excède pas une heure, on parle de phase de repos proprement dit. Lorsqu'elle est de plusieurs heures, on parle alors de phase de stockage ou phase d'attente.
Cette phase de repos permet de stabiliser le mélange obtenu et contenant la composition comprenant au moins une protéine végétale et la composition comprenant au moins une protéine laitière. Indifféremment, on pourra également dans la présente invention parler de phase d'équilibrage.

Le procédé selon l'invention comprend en outre une étape de traitement modifiant la conformation des protéines. Dans la présente description, on désigne par « traitement modifiant la conformation des protéines » tout traitement appliqué à une composition aqueuse comprenant des protéines qui a pour effet de modifier la structure primaire, seconde, tertiaire et/ou quaternaire de ces protéines.

La structure des protéines est la composition en acides aminés et la conformation en trois dimensions des protéines. Elle décrit la position relative des différents atomes qui composent une protéine donnée.

Les protéines se composent d'un enchaînement linéaire d'acides aminés liés par des liaisons peptidiques. Cet enchaînement possède une organisation tridimensionnelle (ou repliement) qui lui est propre. De la séquence au repliement, il existe 4 niveaux de structuration de la protéine.

La structure primaire, ou séquence, d'une protéine correspond à la succession linéaire des acides aminés (ou résidus) la configurant, sans référence à une configuration spatiale. Les protéines sont donc des polymères d'acides aminés. Concrètement, cette structure primaire est représentée par une succession de lettres correspondant aux 20 acides aminés existants.

La structure secondaire décrit le repliement local de la chaîne principale d'une protéine. L'existence de structures secondaires vient du fait que les repliements énergétiques favorables de la chaîne peptidiques sont limités et que seules certaines conformations sont possibles. Ainsi, une protéine peut être décrite par une séquence d'acides aminés mais aussi par un enchaînement d'éléments de structure secondaire. De plus, certaines conformations se trouvent nettement favorisées car stabilisées par des liaisons hydrogènes entre les groupements amide (-NH) et carbonyle (-CO) du squelette peptidique. Il existe trois principales catégories de structures secondaires selon l'échafaudage de liaisons hydrogènes, et donc selon le repliement des liaisons peptidiques : les hélices, les feuillets et les coudes. Il existe des méthodes expérimentales pour déterminer la structure secondaire comme la résonance magnétique nucléaire, le dichroïsme circulaire ou certaines méthodes de spectroscopie infrarouge.

La structure tertiaire d'une protéine correspond au repliement de la chaîne polypeptidique dans l'espace. On parle plus couramment de structure tridimensionnelle. La structure tridimensionnelle d'une protéine est intimement liée à sa fonction : lorsque cette structure est cassée par l'emploi d'un agent dénaturant, la protéine perd sa fonction : elle est dénaturée. La structure tertiaire d'une protéine dépend de sa structure primaire, mais également de son environnement. Les conditions locales qui existent à l'extérieur de chaque compartiment cellulaire, le solvant, la force ionique, la viscosité, la concentration, contribuent à modifier la conformation. Ainsi, une protéine soluble dans l'eau aura besoin d'un environnement aqueux pour adopter sa structure tridimensionnelle.

Enfin, la structure quaternaire des protéines regroupe l'association d'au moins deux chaînes polypeptidiques - identiques ou différentes- par des liaisons non-covalentes, liaisons dîtes faibles (liaisons H, liaison ionique, interactions hydrophobes et force de Van des Waals), mais rarement par des ponts disulfures qui ont pour rôle de créer des liaisons inter chaîne.
Les protéines ont un rôle majeur dans les qualités organoleptiques de nombreux aliments frais ou manufacturés, comme par exemple la consistance et la texture de la viande et produits carnés, du lait et dérivés, des pâtes et du pain. Ces qualités des aliments dépendent très fréquemment de la structure et des propriétés physico-chimiques des composants protéiques ou tout simplement des propriétés fonctionnelles des protéines.
Le terme « propriété fonctionnelle » appliqué aux ingrédients alimentaires est défini comme toute propriété non nutritionnelle qui influence l'utilité d'un ingrédient dans un aliment. Les diverses propriétés vont contribuer pour aboutir aux caractéristiques désirées de l'aliment. Quelques unes des propriétés fonctionnelles des protéines sont : la solubilité, l'hydratation, la viscosité, la coagulation, la texturation, la formation de pâte, les propriétés émulsifiante et moussante.

La conformation d'une protéine est liée à la structure secondaire et tertiaire, elle est réalisée par l'intermédiaire de liaisons faible énergie donc fragiles.

Il existe différents états de transformation des protéines, en fonction du traitement choisi :
- La dénaturation des protéines correspond au passage d'un état ordonné à un état désordonné sans rupture de liaisons covalentes : c'est le déplissement de la protéine ;
- La polymérisation correspond à la formation d'agrégat ;
- La précipitation correspond à la formation d'agrégats de grande taille avec perte totale de la solubilité ;
- La floculation correspond à une agrégation non ordonnée en absence de dénaturation ;
- La coagulation résulte d'une agrégation protéine-protéine avec phénomène de dénaturation ;
- La gélification correspond à une agrégation ordonnée de molécules plus ou moins dénaturées. Il y a formation d'un réseau continu tridimensionnel où les polymères interagissent entre eux et avec le solvant. C'est aussi le résultat de l'équilibre qui existe entre force de cohésion et force de répulsion.

La dénaturation résulte d'une modification des structures quaternaire, tertiaire et secondaire sans fragmentation de la chaîne peptidique. La dénaturation des protéines passe par des structures éphémères qui peuvent aboutir à un dépliement total de la molécule mais on considère aussi que la dénaturation peut résulter d'un accroissement de structure au-delà de la forme native. Le dépliement analogue à une structure en pelote statistique augmente la stabilité des molécules. Cette dénaturation modifie les propriétés des protéines :
- baisse de la solubilité par démasquage de groupes hydrophobes
- diminution des propriétés d'hydratation par modification du pouvoir de rétention d'eau
- perte d'activité biologique
- augmentation de la susceptibilité à la protéolyse
- accroissement de la viscosité intrinsèque
- modification ou inaptitude à la cristallisation

La structure des protéines est très sensible aux traitements physico-chimiques. De nombreux procédés peuvent conduire à la dénaturation des protéines en affectant les structures secondaire, tertiaire et quaternaire. Les traitements physiques pouvant induire une dénaturation sont le chauffage, le refroidissement, les traitements mécaniques, la pression hydrostatique et les radiations ionisantes. Les interactions avec certains produits chimiques peuvent également dénaturer les protéines : acides et bases, métaux et hautes concentrations salines, solvants organiques, etc.

Le traitement modifiant la conformation des protéines peut être choisi dans le groupe constitué par un traitement chimique, un traitement mécanique, un traitement thermique, un traitement enzymatique et la combinaison de plusieurs de ces traitements.

Parmi les traitements chimiques, on peut citer en particulier les traitements modifiant le pH de la composition aqueuse comprenant des protéines et les traitements modifiant la force ionique de la composition aqueuse comprenant des protéines. Dans les traitements chimiques de dénaturation, de nombreux facteurs peuvent intervenir. En premier lieu on peut citer les pH extrêmes qui entraînent un déplissement de la molécule par ionisation de cette dernière et phénomène de répulsion des fragments peptidiques dévoilés. La perte des ions associés à une protéine entraîne la dénaturation des molécules. Les solvants organiques modifient la constante diélectrique du milieu et, par conséquence vont modifier la répartition des charges donc des forces électrostatiques qui maintiennent la cohésion de la structure protéique. Les solvants apolaires peuvent réagir avec les zones hydrophobes et rompre l'interaction hydrophobe qui maintient la conformation de la protéine. Enfin les agents chaotropiques et les agents tensioactifs en cassant soit les liaisons hydrogènes soit les interactions hydrophobes entraînent la dénaturation des protéines.

Parmi les traitements mécaniques appelés également traitement physiques, on peut citer en particulier les traitements d'homogénéisation haute pression de la composition aqueuse comprenant des protéines.

Parmi les traitements thermiques, on peut citer en particulier les traitements de chauffage de la composition aqueuse comprenant des protéines. Les traitements thermiques sont susceptibles de modifier les fonctionnalités de la plupart des ingrédients. Il existe une grande variété de « traitements thermiques » possibles car ceux-ci sont régis par la définition du couple temps-température.

Le traitement thermique peut engendrer de profonde modification comme par exemple la destruction des acides aminés soufrés avec production d'H₂S, de diméthylsulfure, d'acide cystéique (cas des protéines laitières, de la viande, de la chair de poisson...), la destruction de la sérine, de la thréonine, de la lysine. Il peut y avoir des réactions de désamination si la température est supérieure à 100°C. L'ammoniac provient des groupements « acétamido » de la glutamine, de l'asparagine, s'il y a modification des propriétés fonctionnelles (modification du point isoélectrique (pI), apparition de nouvelles liaisons de covalence), il n'y a pas de modification de la valeur nutritionnelle.

Parmi les traitements enzymatiques, on peut citer en particulier l'hydrolyse ménagée et la réticulation. La modification enzymatique des protéines constitue un outil puissant pour améliorer les propriétés technologiques de ces macromolécules. L'hydrolyse par les protéases est une méthode bien connue pour améliorer la solubilité des protéines. Généralement, il est observé que la solubilité augmente avec le degré d'hydrolyse, mais dépend des enzymes utilisées, dont la spécificité détermine la taille et la séquence des peptides libérés. L'aptitude des peptides à former et à stabiliser des mousses et des émulsions dépend aussi des caractéristiques physico-chimiques. Ces peptides doivent présenter une amphiphilicité et avoir une taille minimale (>15-20 résidus) pour former et stabiliser la couche interfaciale. Toutefois, on observe assez souvent des propriétés moussantes plus faibles. Par contre, l'hydrolyse ménagée peut favoriser la production de polypeptides émulsifiants dont la fonctionnalité est supérieure à celle de la propriété d'origine.

A l'inverse des protéases, d'autres enzymes semblent particulièrement intéressantes pour modifier les propriétés fonctionnelles des protéines. Parmi celles-ci, les transglutaminases se sont avérées très performantes. Les transglutaminases sont des transférases qui catalysent la formation d'une liaison * (*-glutamyl)amine entre le groupement carboxyamide d'un résidu glutaminyl d'une protéine et un groupement amine primaire. Si cette fonction est le groupement aminé du résidu lysyl, il y a formation d'une liaison isopeptidique et réticulation des protéines. En l'absence d'amine disponible dans le milieu, l'eau peut jouer le rôle d'accepteur d'acyle et le groupement carboxyamide est alors désamidé. Ainsi, les transglutaminases peuvent induire la réticulation des protéines et permettre la gélification.

Le traitement modifiant la conformation des protéines peut être appliqué à la composition comprenant au moins une protéine végétale, à la composition comprenant au moins une protéine laitière ou à la composition obtenue après le mélange de ces deux compositions.

Le procédé d'obtention de l'assemblage selon l'invention peut comprendre une seule étape de traitement modifiant la conformation des protéines, cette étape de traitement pouvant être appliqué à l'une des deux compositions de protéines avant mélange ou à la composition obtenue après le mélange de ces deux compositions.

Alternativement, le procédé d'obtention de l'assemblage selon l'invention peut comprendre plusieurs étapes de traitement modifiant la conformation des protéines, les traitements pouvant être ou non de même nature, et pouvant être appliqués à des compositions différentes ou successivement à la même composition.

Selon la présente invention, l'étape de traitement modifiant la conformation des protéines consiste à abaisser le pH de la composition comprenant au moins une protéine végétale à une valeur inférieure ou égale à 4, avant le mélange avec la composition comprenant au moins une protéine laitière. De préférence, le pH est abaissé jusqu'à une valeur inférieure ou égale à 3, plus préférentiellement encore inférieure ou égale à 2,5, en particulier compris entre 2 et 2,5.

Cette étape d'abaissement du pH peut être réalisée en ajoutant à la composition végétale aqueuse un acide, et de préférence un acide dont l'utilisation est autorisée dans le domaine agroalimentaire. L'acide peut par exemple être choisi dans le groupe constitué de l'acide chlorhydrique, l'acide acétique, l'acide phosphorique, l'acide citrique, l'acide sorbique, l'acide benzoïque, l'acide tartrique, l'acide lactique, l'acide propionique, l'acide borique, l'acide malique et l'acide fumarique. L'ajout de l'acide peut éventuellement s'accompagner d'une agitation de la composition aqueuse.

La composition acidifiée peut éventuellement être agitée pendant une durée d'au moins 15 minutes, plus préférentiellement d'au moins 30 minutes, plus préférentiellement encore d'au moins 1 heure et en particulier d'au moins 2 heures. Cette agitation favorise avantageusement la dissociation et la solubilisation des protéines végétales dans la composition acidifiée. Cette étape d'agitation peut être réalisée à une température favorisant la dissociation et la solubilisation, de préférence entre 1°C et 100°C, plus préférentiellement entre 2°C et 40°C, et encore plus préférentiellement entre 4°C et 35°C.

Selon un mode de réalisation avantageux de la présente invention, l'étape de traitement modifiant la conformation des protéines consiste à abaisser le pH de la composition comprenant au moins une protéine laitière à une valeur inférieure ou égale à 4, avant le mélange avec la composition comprenant au moins une protéine végétale. Cette étape peut être réalisée comme décrit ci-dessus pour la composition comprenant au moins une protéine végétale.

Avantageusement, ce deuxième mode de réalisation peut être combiné au premier mode de réalisation, le procédé pouvant donc comprendre deux étapes de traitement modifiant la conformation des protéines, l'un appliqué à la composition comprenant au moins une protéine végétale, l'autre appliqué à la composition comprenant au moins une protéine laitière, les deux traitements consistant à abaisser le pH de la composition à une valeur inférieure ou égale à 4.

Lorsque le procédé selon l'invention comprend une étape de traitement consistant à abaisser le pH de la composition, il peut avantageusement comprendre, en outre, une étape de remontée du pH de la composition obtenue après mélange à une valeur comprise entre 5 et 8. De préférence, le pH est remonté jusqu'à une valeur comprise entre 5,5 et 7,5, plus préférentiellement encore jusqu'à une valeur comprise entre 6 et 7.

Cette étape de remontée du pH peut être réalisée en ajoutant au mélange un alkali, de préférence un alkali dont l'utilisation est autorisée dans le domaine agroalimentaire. La base peut par exemple être choisie dans le groupe constitué de la soude, le sorbate de sodium, le sorbate de potassium, le sorbate de calcium, le benzoate de sodium, le benzoate de potassium, le benzoate de potassium, le formiate de sodium, le formiate de calcium, le nitrate de sodium, le nitrate de potassium, l'acétate de potassium, le diacétate de potassium, l'acétate de calcium, le diacétate de potassium, l'acétate de calcium, l'acétate d'ammonium, le propionate de sodium, le propionate de calsium et le propionate de potassium. L'ajout de la base peut éventuellement s'accompagner d'une agitation du mélange, pendant une durée d'au moins 15 minutes, plus préférentiellement d'au moins 30 minutes, plus préférentiellement encore d'au moins 1 heure et en particulier d'au moins 2 heures.
Cette étape d'agitation peut être réalisée à une température favorisant la dissociation et la solubilisation, de préférence entre 1°C et 100°C, plus préférentiellement entre 2°C et 40°C, et encore plus préférentiellement entre 4°C et 35°C.

Selon un mode de réalisation avantageux de la présente invention, l'étape de traitement modifiant la conformation des protéines consiste en une étape d'homogénéisation de la composition obtenue après mélange. On a constaté que cette étape d'homogénéisation permettait avantageusement d'obtenir une meilleure solubilisation des protéines végétales et de favoriser les interactions entre les protéines végétales et les protéines laitières.

L'homogénéisation peut être réalisée selon des techniques connues de l'homme du métier. Une technique particulièrement préférée est l'homogénéisation à haute pression. Il s'agit d'un traitement physique au cours duquel un produit liquide ou pâteux est projeté sous forte pression à travers une tête d'homogénéisation de géométrie particulière. Ce traitement se traduit par une réduction de la taille des particules solides ou liquides se trouvant sous forme dispersée dans le produit traité. La pression de l'homogénéisation haute pression est typiquement comprise entre 30 bars et 1 000 bars. Dans le procédé objet de la présente invention, cette pression est de préférence comprise entre 150 bars et 500 bars, plus préférentiellement entre 200 bars et 400 bars, et encore plus préférentiellement entre 250 bars et 350 bars. En outre, un ou plusieurs cycles d'homogénéisation peuvent être effectués. De préférence, le nombre de cycles d'homogénéisation à haute pression est compris entre 1 et 4.

L'homogénéisation peut également être réalisée à l'aide d'autres dispositifs connus, par exemple choisis parmi les mixers, les broyeurs colloïdaux, les homogénéisateurs à meules et à microbilles, les homogénéisateurs ultrasoniques et les homogénéisateurs à obturateurs.

Le procédé de production de l'assemblage selon l'invention peut comprendre plusieurs étapes d'homogénéisation de la composition obtenue après mélange. En particulier, une première étape d'homogénéisation peut être appliquée sur la composition obtenue après mélange ayant préalablement subi une étape de traitement consistant à abaisser le pH de la composition, puis une seconde étape d'homogénéisation peut être appliquée sur la composition après une étape de remontée du pH.

Avantageusement, ce troisième mode de réalisation peut être combiné au premier ou au deuxième mode de réalisation, le procédé pouvant donc comprendre deux étapes de traitement modifiant la conformation des protéines, l'un appliqué à la composition comprenant au moins une protéine végétale ou à la composition comprenant au moins une protéine laitière, consistant à abaisser le pH de la composition à une valeur inférieure ou égale à 4, et l'autre consistant à homogénéiser la composition obtenue après mélange.

En particulier, le procédé d'obtention d'un assemblage d'au moins une protéine végétale et d'au moins une protéine laitière peut comprendre les étapes consistant à :
- se munir d'une composition aqueuse comprenant au moins une protéine végétale ;
- abaisser le pH de ladite composition à une valeur inférieure ou égale à 4 pour obtenir une composition acidifiée ;
- introduire au moins une protéine laitière dans ladite composition acidifiée pour obtenir un mélange ;
- homogénéiser le mélange obtenu ;
- remonter le pH dudit mélange homogénéisé à une valeur comprise entre 5 et 8 pour obtenir ledit assemblage.

Le procédé objet de la présente invention permet d'obtenir une composition aqueuse comprenant un assemblage d'au moins une protéine végétale et d'au moins une protéine laitière, qui est également un objet de la présente invention.

Il a en effet été constaté que le procédé de préparation décrit ci-dessus, et en particulier la présence d'une étape de traitement modifiant la conformation des protéines, favorise la formation d'assemblages entre la protéine végétale et la protéine laitière.

L'assemblage d'au moins une protéine végétale et d'au moins une protéine laitière ainsi obtenu, qui est également un objet de la présente invention, se distingue du simple mélange physique de ces deux types de protéines. Il s'agit d'une nouvelle structure à l'échelle supramoléculaire.

Ledit assemblage peut se présenter sous la forme d'une composition aqueuse, d'une composition aqueuse concentrée ou d'une poudre. Dans le cas d'une composition aqueuse, on parle plutôt de dispersion aqueuse.

Une composition aqueuse, ou dispersion aqueuse, comprenant l'assemblage d'au moins une protéine végétale et d'au moins une protéine laitière est obtenue à l'issue du procédé objet de la présente invention. Cette composition ou dispersion aqueuse a un pH compris de préférence entre 5 et 8 plus préférentiellement entre 5,5 et 7,5, et encore plus préférentiellement entre 5,8 et 7,1.

La teneur totale en protéines de la composition est de préférence comprise entre 20 % et 100 % en poids de produit sec, plus préférentiellement comprise entre 30 % et 90%, encore plus préférentiellement comprise entre 35 % et 85 %, et en particulier comprise entre 40 % et 80 %.

Lesdites teneurs étant indiquées en pourcentage de poids de produit sur le poids sec de la composition.

Selon un autre mode de réalisation, la teneur en protéines de la composition est comprise entre 50% et 90% en poids sur produit sec.

Lorsqu'on est en présence d'une dispersion aqueuse, c'est-à-dire lorsque l'assemblage est mis en suspension dans un liquide, la teneur en protéines est indiquée en concentration massique, c'est-à-dire en concentration pondérale qui exprime le rapport entre la masse d'un soluté, i.e. les protéines et le volume de dispersion aqueuse.

Ledit assemblage comprenant au moins une protéine végétale et au moins une protéine laitière peut éventuellement comprendre d'autres ingrédients. Ces ingrédients optionnels peuvent avoir des propriétés intéressantes pour certaines applications. Ils peuvent être choisis dans le groupe constitué par les fibres solubles, les fibres insolubles, les vitamines, les sels minéraux, les oligo-éléments et leurs mélanges. Les ingrédients optionnels peuvent être apportés par les compositions comprenant au moins une protéine végétale ou au moins une protéine laitière, ou ils peuvent être ajoutés lors de la préparation de l'assemblage.

Selon un mode de réalisation préférentiel de la présente invention, ledit assemblage comprenant au moins une protéine végétale et au moins une protéine laitière inclut une fibre végétale soluble.

De préférence, ladite fibre soluble d'origine végétale est choisie dans le groupe constitué par les fructanes dont les Fructo-oligosaccharides (FOS) et l'inuline, les Gluco-oligosaccharides (GOS), les Isomalto-oligosaccharides (IMOs), les Trans-galacto-oligosaccharide (TOS), les pyrodextrines, le polydextrose, les maltodextrines branchées, les dextrines indigestibles et les oligosaccharides solubles issus de plantes oléagineuses ou protéagineuses.

Par fibre soluble, on entend des fibres solubles dans l'eau. Les fibres peuvent être dosées selon différentes méthodes AOAC. On peut citer à titre d'exemple, les méthodes AOAC 997.08 et 999.03 pour les fructanes, les FOS et l'inuline, la méthode AOAC 2000.11 pour le polydextrose, la méthode AOAC 2001.03 pour le dosage des fibres contenues dans les maltodextrines branchées et les dextrines indigestibles ou la méthode AOAC 2001.02 pour les GOS ainsi que les oligosaccharides solubles issus de plantes oléagineuses ou protéagineuses.

Selon un mode de réalisation particulièrement avantageux de la présente invention, ledit assemblage comprend des fibres végétales solubles qui sont des maltodextrines branchées.

On entend par maltodextrines branchées (MDB), les maltodextrines spécifiques identiques à celles décrites dans le brevet EP 1.006.128-B1 dont la Demanderesse est titulaire. Ces MDB ont l'avantage de représenter une source de fibres indigestibles bénéfiques pour le métabolisme et pour l'équilibre intestinal. En particulier, on pourra utiliser des MDB présentant entre 15 et 35% de liaisons glucosidiques 1-6, une teneur en sucres réducteurs inférieure à 20%, une masse moléculaire moyenne en poids MW comprise entre 4000 et 6000 g/mole et une masse moléculaire moyenne en nombre Mn comprise entre 250 et 4500 g/mole.

Certaines sous-familles de MDB décrites dans la susdite demande peuvent aussi être utilisées conformément à l'invention. Il s'agit par exemple de MDB de hauts poids moléculaires présentant une teneur en sucres réducteurs au plus égale à 5 et un Mn compris entre 2000 et 4500 g/mole. Les MDB de bas poids moléculaires présentant une teneur en sucres réducteurs comprise entre 5 et 20% et une masse moléculaire Mn inférieure à 2000 g/mole peuvent également être employées.

L'utilisation de NUTRIOSE®, qui est une gamme complète de fibres solubles, reconnues pour leurs bienfaits, et fabriquées et commercialisées par l'une des Demanderesses est particulièrement intéressante. Les produits de la gamme NUTRIOSE® sont des dérivés d'amidon de blé ou de maïs partiellement hydrolysés, qui contiennent jusqu'à 85 % de fibre totale. Cette richesse en fibre permet d'augmenter la tolérance digestive, d'améliorer la gestion de calorie, de prolonger le dégagement d'énergie et d'obtenir un taux de sucre inférieur. De plus, la gamme NUTRIOSE® est l'une des compositions de fibres les mieux tolérées disponibles sur le marché. Elle montre une tolérance digestive plus élevée, permettant une meilleure incorporation que d'autres fibres, ce qui représente de vrais avantages alimentaires.

Selon un mode de réalisation de l'invention, le procédé d'obtention dudit assemblage comprend en outre une étape consistant à faire subir à la composition aqueuse comprenant un assemblage d'au moins une protéine végétale et d'au moins une protéine laitière un traitement thermique à température élevée et pendant un temps court, ledit traitement pouvant être choisi parmi les traitements HTST (*High Temperature Short Time*) et UHT (Ultra Haute Température). Cette étape optionnelle permet avantageusement de réduire les risques de contamination bactériologique et d'améliorer les propriétés de conservation de la composition.

La composition aqueuse comprenant l'assemblage selon la présente invention peut éventuellement être concentrée. Le procédé objet de l'invention peut donc en outre comprendre une étape de concentration de ladite composition. Cette étape de concentration peut intervenir éventuellement après une étape de traitement thermique et/ou une étape de stabilisation.

Après concentration, la teneur totale en protéines de la composition concentrée est de préférence comprise entre 100 g/kg et 600 g/kg en poids de protéines sur le poids total de la composition, plus préférentiellement comprise entre 150 g/kg et 400 g/kg et en particulier comprise entre 200 g/kg et 300 g/kg.

Le procédé objet de l'invention peut en outre comprendre une étape consistant à sécher la composition aqueuse, éventuellement concentrée, comprenant l'assemblage d'au moins une protéine végétale et d'au moins une protéine laitière.

Le procédé de séchage peut être choisi parmi les techniques connues de l'homme du métier, et en particulier dans le groupe constitué par l'atomisation, l'extrusion et la lyophilisation, la granulation, le lit fluidisé, les rouleaux sous vide, la micronisation.

Les conditions opératoires de l'étape de séchage sont adaptées à l'équipement choisi, de façon à permettre l'obtention d'une poudre.

L'atomisation est une opération unitaire de séchage qui consiste à transformer en poudre un liquide pulvérisé sous forme de gouttelettes mises en contact avec un gaz chaud. Les conditions de la pulvérisation déterminent la taille des gouttelettes produites, leur trajectoire, leur vitesse et par conséquent la dimension finale des particules sèches, ainsi que les propriétés des poudres obtenues : écoulement, caractère instant lié à leur solubilité, densité, comprimabilité, friabilité, etc. L'étape d'atomisation peut être réalisée dans un atomiseur ou une tour d'atomisation, dans laquelle la composition liquide à sécher est pulvérisée dans un courant de gaz chaud. Ce gaz chaud apporte les calories nécessaires à l'évaporation du solvant de la composition et absorbe, pour l'évacuer, l'humidité libérée par le produit en cours de séchage. La composition liquide est admise au sommet par une buse ou une turbine, la poudre « atomisée » obtenue est récoltée à la base de la tour. Le solide sec est séparé du gaz d'atomisation par un (ou des) cyclone(s), ou par filtration (filtre à manches par exemple). Dans certains cas, si cela s'avère nécessaire, la tour peut être remplie d'un gaz inerte pour éviter les phénomènes d'oxydation.

L'extrusion est un procédé dans lequel un matériau est contraint de traverser une filière ayant la section de la pièce à obtenir. Les paramètres de températures sont sélectionnés aisément par l'homme du métier en fonction de la teneur en eau de la composition avant séchage. La composition extrudée peut ensuite être successivement soumise à un refroidissement, à un broyage, et éventuellement un tamisage pour obtenir une poudre.

La lyophilisation, ou cryodessiccation, consiste à ôter l'eau de la composition en soumettant celle-ci à une phase de surgélation, puis à une phase de chauffage à très basse pression pour provoquer la sublimation du solvant de la composition.

L'assemblage d'au moins une protéine végétale et d'au moins une protéine laitière selon l'invention peut se présenter sous la forme d'une poudre.

La taille moyenne de la poudre obtenue peut être caractérisée par son diamètre moyen volumique (moyenne arithmétique) D 4,3, appelé encore diamètre moyen volumique laser D4,3. Il est compris de préférence entre 10µm et 500µm, de préférence entre 30µm et 350µm et encore plus préférentiellement entre 50µm et 200µm.

Selon un mode préférentiel, le diamètre moyen volumique D 4,3 de la dite poudre granulée est compris entre 60µm et 120µm.

Selon un mode particulier de la présente invention, 90% de la poudre a un diamètre inférieure à 1000µm, de préférence inférieur à 500µm, et plus préférentiellement encore inférieur à 400µm. En particulier, 90% de la poudre a un diamètre inférieur à 370µm. Cette valeur correspond au d₉₀.

Selon un autre mode particulier de la présente invention, 50% de la poudre a un diamètre inférieur à 500µm, de préférence inférieur à 300µm, et plus préférentiellement encore inférieur à 250µm. En particulier, 50% de la poudre a un diamètre inférieur à 220µm. Cette valeur correspond au d₅₀.

Selon un autre mode particulier de la présente invention, 10% de la poudre a un diamètre inférieur à 300µm, de préférence inférieur à 200µm, et plus préférentiellement encore inférieur à 150µm. En particulier, 10% de la poudre a un diamètre inférieur à 100µm. Cette valeur correspond au d₁₀.

Ces mesures de granulométrie, notamment le diamètre moyen volumique D 4,3 et les trois valeurs d₉₀, d₅₀ et d₁₀ sont déterminées sur un granulomètre à diffraction LASER type LS 230 de la société BECKMAN-COULTER, équipé de son module de dispersion poudre (voie sèche), en suivant le manuel technique et les spécifications du constructeur. La gamme de mesure du granulomètre à diffraction LASER type LS 230 est de 1 µm à 2 000 µm.

L'assemblage objet de l'invention possède des propriétés fonctionnelles et/ou sensorielles différentes de celles du simple mélange physique de protéines végétales et de protéines laitières. En particulier, cet assemblage possède au moins l'une des propriétés fonctionnelles suivante :
- une solubilité améliorée ;
- une amélioration de la tenue en suspension ;
- un pouvoir coagulant amélioré ;
par rapport au simple mélange physique de protéines végétales et de protéines laitières. Avec l'assemblage selon l'invention, on observe par conséquent un effet synergique sur les propriétés fonctionnelles.

En outre, l'assemblage selon l'invention peut posséder des propriétés fonctionnelles intéressantes, en particulier :
- un pouvoir émulsifiant ;
- un pouvoir moussant ;
- un pouvoir gélifiant ;
- un pouvoir épaississant,
- un pouvoir viscosant,
- un pouvoir foisonnant ;
- un pouvoir mouillant (capacité d'absorption d'eau) ;
- un pouvoir filmogène et/ou adhésif
- un pouvoir de réactivité thermique,
- un pouvoir dans les réactions de Maillard.

Il existe un lien entre les propriétés fonctionnelles et les propriétés sensorielles. La synergie mise en évidence sur les propriétés fonctionnelles se retrouve donc également sur les propriétés sensorielles de l'assemblage selon l'invention.

La synergie reflète communément un phénomène par lequel plusieurs acteurs, facteurs ou influences agissant ensemble créent un effet plus grand que la somme des effets attendus s'ils avaient opéré indépendamment, ou créent un effet que chacun d'entre eux n'aurait pas pu obtenir en agissant isolément. Dans la présente demande, le mot est est également utilisé pour désigner un résultat plus favorable lorsque plusieurs éléments d'un système agissent de concert.

Dans le cadre de la présente invention, la synergie reflète l'existence d'un mélange intime entre les différents composants de l'assemblage, que leur répartition au sein de l'assemblage est sensiblement homogène, et que ceux-ci ne sont pas uniquement liés entre eux par un simple mélange physique.

Les Sociétés Demanderesses ont constaté qu'un mélange d'au moins une protéine laitière et d'au moins une protéine végétale, dont les caractéristiques physiques ont été modifiées en employant un procédé approprié, de telle sorte que l'on obtienne simultanément des propriétés fonctionnelles très intéressantes ne pouvant être obtenues si chaque composé est utilisé séparément ou si les composés sont utilisés simultanément mais sous la forme d'un simple mélange des différents constituants.

Des propriétés fonctionnelles très intéressantes conférées par ledit assemblage concernent ses pouvoirs émulsifiant, moussant et gélifiant, en comparaison au simple mélange des constituants de cet assemblage.

Les propriétés émulsifiantes sont dues à la faculté de réduire les tensions interfaciales entre composants hydrophiles et hydrophobes d'un aliment. Elles sont directement liées à la solubilité de la protéine. Les poudres possédant ces propriétés de surface auront un potentiel d'utilisation important dans les émulsions en général, dans les poudres de lait, réengraissées ou non, ainsi que dans les aliments contenant eau et graisses (charcuterie, viande, condiment).

Ainsi, une des utilisations avantageuses de l'assemblage selon la présente invention ou susceptible d'être obtenue selon la mise en oeuvre du procédé de préparation de l'assemblage selon l'invention tel que décrit ci-dessus, est qu'il peut-être utilisé comme agent émulsifiant dans les compositions précédemment citées, en substitution totale de tout autre agent émulsifiant, et en particulier de la lécithine. Ledit assemblage peut être lui-même totalement exempt d'agent émulsifiant, considéré comme des additifs selon la règlementation européenne. D'ailleurs, une des utilisations avantageuses de l'assemblage selon la présente invention ou susceptible d'être obtenue selon la mise en oeuvre du procédé de préparation de l'assemblage selon l'invention tel que décrit ci-dessous, est qu'il peut-être utilisé comme agent émulsifiant dans les compositions précédemment citées, en substitution totale de tout autre agent émulsifiant, et en particulier de la lécithine.

En effet, l'utilisation dudit assemblage permet d'éliminer totalement la lécithine des formulations alimentaires, et plus particulièrement des formulations aimentaires se présentant totalement ou partiellement sous la forme d'une émulsion, c'est-à-dire contenant au moins deux ingrédients non miscibles (typiquement de l'eu et de l'huile).

De façon générale, les émulsifiants, appelés parfois émulsionnants, stabilisent les émulsions. Les émulsifiants actuellement utilisés dans les industries sont soit des produits naturels purifiés, soit des produits chimiques synthétiques dont les structures sont très proches de celles des produits naturels.

Ce sont le plus souvent des tensioactifs ou agents de surface. Ce sont des molécules qui possèdent une extrémité ayant une affinité pour l'eau (hydrophile) et une extrémité ayant une affinité pour l'huile (hydrophobe). Dans l'agroalimentaire, les émulsifiants sont utilisés pour augmenter l'onctuosité de certains produits, permettant d'obtenir une texture particulière. Un des émulsifiants les plus connu est incontestablement la lécithine.

En effet, traditionnellement la lécithine, connue également sous le nom de phosphatidylcholine, est utilisée comme émulsifiant dans les industries alimentaires, cosmétiques et autres. C'est un émulsifiant naturel que l'on tire industriellement par un traitement aqueux de l'huile de soja. Elle se présente sous la forme d'un liquide pâteux, de couleur brune. Son aspect est peu appétissant et pas très agréable au goût. La lécithine se classe dans la catégorie des lipides. Elle peut également être extraite des jaunes d'oeufs mais le procédé est trop couteux pour être appliqué à un stade industriel.

Les lécithines sont des additifs alimentaires, et sont soumises, comme les autres additifs alimentaires, à une règlementation européenne stricte qui régit l'évaluation de leur innocuité, leur autorisation et leur étiquetage. Ces règlementations exigent que tous les émulsifiants ajoutés, sous quelque forme que ce soit, soient mentionnés sur l'emballage du produit soit par leur nom, soit par leur code européen (lettre E suivie d'un nombre, E322 pour la lécithine) comme tous les autres additifs alimentaires. Qui plus est, comme les lécithines sont extraites du soja pour une utilisation au stade industriel, elles ont également subi le contrecoup de l'image négative véhiculée par les Organismes Génétiquement Modifiés dont le soja peut faire parti.

Ainsi l'assemblage selon la présente invention ou susceptible d'être obtenue selon la mise en oeuvre du procédé de préparation de l'assemblage selon l'invention tel que décrit ci-dessus, étant de préférence lui-même dénué d'émulsifiants tels que la lécithine, permet d'éviter l'utilisation d'autres émulsifiants, et en particulier de la lécithine, et permet ainsi de s'affranchir à la fois des risques d'allergies et de l'image négative liés au soja ainsi que l'étiquetage sur l'emballage de la lécithine comme additif alimentaire.

Les propriétés moussantes, qui sont très appréciées en pâtisseries (cakes, soufflets, meringues) et dans la fabrication de mousses, laitières ou autres, de crèmes fouettées, résultent d'un déplissement partiel des protéines qui s'orientent à l'interface eau/air. L'assemblage possède un excellent pouvoir moussant, extrêmement stable en fonction du temps.

Une autre propriété très intéressante conférée par ledit assemblage selon la présente invention est la nette amélioration du goût d'une part, de la palatabilité et du corps défini également par la viscosité en bouche d'autre part. En effet, l'assemblage possède un goût neutre, contrairement au simple mélange des deux constituants qui sont au moins une protéine laitière et au moins une protéine végétale, qui peut lui présenter un goût de légumineuse plus marqué et être par conséquent un frein à certaines applications alimentaires. Dans certaines applications, la palatabilité et le corps se trouvent également améliorés par rapport au simple mélange.

Ces propriétés fonctionnelles très intéressantes résultant de la réelle synergie entre les constituants de l'assemblage et n'existant pas sur de simples mélanges les destinent entre autre à des applications très diversifiées et variées.

Un autre aspect de la présente invention concerne l'utilisation de l'assemblage d'au moins une protéine végétale et d'au moins une protéine laitière selon l'invention dans les domaines de la cosmétique, de la détergence, de l'agrochimie, des formulations industrielles, pharmaceutiques, des matériaux de construction, des fluides de forage, en fermentation, en nutrition animale et dans le domaine agroalimentaire. L'utilisation dans le domaine agroalimentaire est particulièrement préférée.

Par conséquent, la présente invention concerne également des compositions cosmétique, détergente, agrochimique, des formulations industrielles, pharmaceutiques, des matériaux de construction, des fluides de forage, des milieux de fermentation, des compositions nutritionnelles animales, des applications alimentaires comprenant l'assemblage selon la présente invention ou susceptible d'être obtenue selon la mise en oeuvre du procédé objet de la présente invention.

Dans ces domaines, l'assemblage selon l'invention peut être utilisé en tant qu'agent fonctionnel, et en particulier comme :
- agent émulsifiant,
- agent moussant,
- agent gélifiant,
- agent épaississant,
- agent viscosant,
- agent foisonnant,
- agent rétenteur d'eau,
- agent filmogène et/ou adhésif
- agent ayant un pouvoir dans les réactions de Maillard.

Par conséquent, la présente invention concerne également un agent émulsifiant, moussant, gélifiant, viscosant, foisonnant, rétenteur d'eau et/ou un réactif thermique (c'est-à-dire un agent ayant un pouvoir dans les réactions de Maillard), comprenant l'assemblage selon la présente invention ou susceptible d'être obtenu par le procédé objet de la présente invention.

L'invention s'étend ainsi en particulier aux formulations alimentaires comprenant l'assemblage selon l'invention choisies dans le groupe constitué par :
- les boissons,
- les produits laitiers (dont par exemple les fromages frais et affinés, les fromages fondus éventuellement tartinables, les laits fermentés, les smoothies au lait, les crèmes fouettées, les crèmes fermentées, les mousses, les produits foisonnés, les yaourts, les spécialités laitières, les glaces fabriquées à partir de lait),
- les desserts lactés,
- les préparations destinées à la nutrition clinique et/ou à des individus souffrant de dénutrition,
- les préparations destinées à la nutrition infantile,
- les mélanges de poudres destinés à des produits de régime, ou pour sportifs,
- les produits hyperprotéinés pour la nutrition diététique ou particulière,
- les soupes, sauces, et aides culinaires,
- les confiseries, comme le chocolat par exemple et tous les produits dérivés de ce dernier,
- les produits à base de viande, plus particulièrement dans les secteurs des pâtes fines et des saumures, notamment dans la fabrication de jambons et des charcuteries,
- les produits à base de poissons, comme les produits à base de surimi,
- les produits céréaliers comme le pain, les pâtes, biscuits, pâtisseries, céréales et barres,
- les produits végétariens et plats cuisinés,
- les produits fermentés à base de protéines végétales, comme le tofu par exemple,
- les agents blanchissants tels les coffee whiteners,
- les produits destinés à l'alimentation animale, comme par exemple les produits destinés à l'alimentation des veaux.

Une des utilisations particulièrement avantageuse et intéressante de la présente invention concerne la réalisation d'un produit laitier choisi dans le groupe constitué par les fromages frais et affinés, les fromages tartinables, les laits fermentés, les smoothies au lait, les yaourts, les spécialités laitières, les glaces fabriquées à partir de lait.

Selon un autre mode plus préférentiel, l'assemblage selon l'invention est utilisé pour la fabrication de fromages.

Dans la présente invention, le terme fromage désigne un aliment obtenu à partir de lait coagulé ou de produits laitiers, comme la crème, puis éventuellement d'un égouttage, suivi ou non d'une étape de fermentation et éventuellement d'un affinage (fromages affinés). Selon le décret français n° 2007-628 du 27 avril 2007, la dénomination « fromage » est réservée au produit fermenté ou non, affiné ou non, obtenu à partir de matières d'origine exclusivement laitière (lait entier, lait partiellement ou totalement écrémé, crème, matière grasse, babeurre), utilisées seules ou en mélange, et coagulées en totalité ou en partie avant égouttage ou après élimination partielle de leur eau.

Dans la présente invention, le terme fromage désigne également tous les fromages fondus et tous les fromages fondus tartinables. Ces deux types de fromages sont obtenus par broyage, mélange, fonte et émulsification, sous l'effet de la chaleur et d'agents émulsifiants, d'une ou plusieurs variétés de fromage, avec ou sans adjonction de constituants laitiers et/ou d'autres denrées alimentaires (crème, vinaigre, épices, enzymes,..).

Dans un autre mode préférentiel, l'assemblage selon l'invention est utilisé pour la fabrication de yaourts ou laits fermentés.

L'invention sera encore mieux comprise à la lecture des exemples qui suivent, lesquels se veulent illustratifs en faisant seulement état de certains modes de réalisation et de certaines propriétés avantageuses selon l'invention, et non limitatifs.

### EXEMPLES

### Exemple 1 : Réalisation des assemblages protéiques

### A. Matières premières

**Protéines laitières** : Les protéines laitières utilisées sont issues d'une fraction de lait et contiennent 92% de caséines micellaires sur la matière azotée totale. Ce lot est dénommé rétentat de caséines micellaires PROMILK 852 B commercialisé par la société INGREDIA, et est sous la forme liquide (rétentat à 15 % de matière sèche), stabilisé par addition de 0.02% de bronopol (agent de conservation), et conservé à 4°C.

**Protéines végétales** : Les exemples ont été réalisés avec trois lots différents de protéines de pois.
- Un lot de protéines de pois obtenus par ultrafiltration dénommé UF. Ce lot a été obtenu en passant un extrait liquide de NUTRALYS® S85 M commercialisé par la société ROQUETTE frères sur une membrane de seuil de coupure de 50 KD. Les protéines purifiées obtenues ont été concentrées par diafiltration selon les techniques classiques jusqu'à l'obtention d'une solution concentrée à 75% en protéines. Enfin, le concentré protéique a été lyophilisé et la poudre obtenue a été conservée à 4°C.
- Une poudre de protéines de pois NUTRALYS® S85 M commercialisé par la société ROQUETTE frères possédant un taux de 85% de protéines totales.
- Un floculat de protéines de pois obtenu par précipitation isoélectrique (pH 4,5) du NUTRALYS® S85 M. le floculat est non séché, mais stabilisé par addition d'azoture de sodium à 0,02%.

### B. Les procédés de mélange : constitution des assemblages

La solubilisation des protéines de pois est une étape importante car ce sont des protéines globulaires solubles à des ph extrêmes (pH ≤ 3 et ≥ 7). La solubilisation de ces protéines permet leurs interactions avec les caséines, et l'obtention d'un mélange vraiment intime expliquant la synergie observée dans les propriétés fonctionnelles.

Pour les différents assemblages réalisés entre les protéines de pois et les protéines laitières, le taux de protéines final des assemblages protéiques est de 4%, soit 40 mg/ml de protéines totales dans l'assemblage.

### Trois stratégies d'assemblage ont été testées.

### a. Stratégie d'assemblage à pH 7

- Préparation d'une solution de 16mg/ml de poudre de protéines de pois dans de l'eau. Aucune modification de pH n'est à faire. Le pH obtenu est de 6,9-7. Agitation à 500 rpm avec un barreau magnétique pendant 2 h.
- Préparation d'une solution de 64 mg/ml de poudres de protéines laitières PROMILK 852 B Aucune modification de pH n'est à faire. Le pH obtenu est de 7.
- Mélange des protéines de pois aux protéines laitières 50/50 (v/v).
- Agitation à 550 rpm pendant 1 heure à température ambiante.
- pH final obtenu est de 6,9 - 7.
- Homogénéisation par deux passages à 300 bars à température ambiante.

### b. Stratégie d'assemblage en abaissant le pH à 5,8 - 6

- Préparation d'une solution de 16mg/ml de poudre de protéines de pois dans de l'eau.
- Abaissement du pH à 2.5 avec de l'HCl 1N sous agitation à 500 rpm.
- Solubilisation sous agitation à 500 rpm avec un barreau magnétique à 4°C pendant 2 heures.
- Préparation d'une solution de 64 mg/ml de poudres de protéines laitières PROMILK 852 B. Aucune modification de pH n'est à faire. Le pH obtenu est de 7.
- Verser la solution de protéines de pois dans la solution de protéines laitières dans un ratio 50/50. Le mélange se fait au goutte à goutte sous agitation à 1000 rpm sous contrôle du pH-mètre pour ne pas descendre au-dessous d'un pH de 5.3. Selon la nature de protéines de pois (UF, Nutralys ou floculat), le pH final du mélange est entre 5.8 et 6.
- Homogénéisation par deux passages à 300 bars à température ambiante.

### c. Stratégie d'assemblage en abaissant le pH à 5,8 - 6, puis en le remontant à 6,9

- Préparation d'une solution de 16mg/ml de poudre de protéines de pois dans de l'eau.
- Abaissement du pH à 2.5 avec de l'HCl 1N sous agitation à 500 rpm.
- Solubilisation sous agitation à 500 rpm avec un barreau magnétique à 4°C pendant 2 heures.
- Préparation d'une solution de 64 mg/ml de poudres de protéines laitières PROMILK 852 B. Aucune modification de pH n'est à faire. Le pH obtenu est de 7.
- Verser la solution de protéines de pois dans la solution de protéines laitières dans un ratio 50/50. Le mélange se fait au goutte à goutte sous agitation à 1000 rpm sous contrôle du pH-mètre pour ne pas descendre au-dessous d'un pH de 5.3. Selon la nature de protéines de pois (UF, Nutralys ou floculat), le pH final du mélange est entre 5.8 et 6.
- Remontée du pH des assemblages à 6.9 avec de la soude 1N
- Homogénéisation par deux passages à 300 bars à température ambiante.

### C. Analyse des assemblages protéiques

Afin de pouvoir réaliser les différentes analyses sur les matières premières d'une part et les assemblages d'autre part, il a été nécessaire de centrifuger les différents échantillons et de ne travailler que sur la fraction soluble.
- Centrifugation des solutions protéiques et des assemblages à 15000 g pendant 30 min à 20°C.
- Filtration du surnageant à travers un filtre en cellulose avec des ouvertures de pores de 0, 45µm.

### Exemple 2 Détermination du taux de protéines

Pour déterminer le taux de protéines dans les différents échantillons, on peut effectuer le dosage de la fraction azotée soluble contenue dans l'échantillon selon la méthode de KJELDAHL (NF V03-050, 1970)., La détermination de l'azote ammoniacal est basée sur la formation d'un complexe coloré entre l'ion ammonium, le salicylate de sodium et du chlore, dont l'intensité de coloration est mesurée à 660 nm. Cette méthode est effectuée avec un appareil automatique à flux liquide continu TECHNICON.

La teneur en protéines des échantillons est estimée en multipliant leur teneur en azote par le facteur de conversion 6,25.

Cette méthode est bien connue de l'homme du métier.

Pour déterminer le taux de protéines solubles, on mesure la teneur en protéines solubles dans l'eau dont le pH est ajusté à 7,5 +/- 0,1 à l'aide d'une solution de HCl ou NaOH, par une méthode de dispersion d'une prise d'essai de l'échantillon dans de l'eau distillée, centrifugation et analyse du surnageant. Dans un bécher de 400 ml, on introduit 200,0 g d'eau distillée à 20°C +/- 2 °C, et on place le tout sous agitation magnétique (barreau aimanté et rotation à 200 tr/min). On ajoute exactement 5 g de l'échantillon à analyser. On agite pendant 30 min, et on centrifuge pendant 15 min à 4 000 tr/min. On réalise, sur le surnageant la méthode de détermination de la teneur en azote selon la méthode précédemment décrite.

### Exemple 3 : Résultats de la stratégie de l'abaissement du pH

L'abaissement du pH (2,5) tel que décrit dans l'exemple 1 précédent a pour objectif de solubiliser les protéines de pois afin de changer leur conformation et de provoquer leur dépliement de manière à ce que leurs sites hydrophobes soient exposés au solvant et puissent interagir avec les protéines laitières.

Le tableau 1 ci-dessous présente les fractions solubles et insolubles des protéines de pois en fonction du pH

**TABLEAU 1**

| Protéines de pois | pH 7 | pH 2.5 remonté à 7 |
|---|---|---|
| Concentration totale (mg/ml) | 16 | 16 |
| Fraction soluble (mg/ml) | 7,5 | 10,7 |
| Fraction insoluble (mg/ml) | 8,5 | 5,3 |

Le dosage des protéines solubles dans les solutions protéiques de pois montre une amélioration de la solubilité avec l'abaissement du pH. En effet, la solution de 16 mg/ml présente une fraction soluble de 7.5 mg/ml à pH 7 alors qu'on solubilise jusqu'à 10.7 mg/ml en abaissant le pH à 2.5 et en le remontant à 7.

### Exemple 4 : Détermination de la stabilité des assemblages protéiques obtenus

La stabilité des protéines dans les matières premières de départ aux différents pH a été appréciée puis comparée au comportement de ces mêmes protéines dans les assemblages. Cette stabilité physique a été évaluée par la mesure :
- du maintien en suspension des protéines,
- de la solubilité des protéines,
- de la dispersibilité des protéines.

A chaque fois les mesures ont été réalisées sur les matières premières de départ (protéines de pois à 8 mg/ml et protéines laitières à 32 mg/ml), ainsi que sur les assemblages obtenus selon l'exemple 1.

### A. La mesure du maintien en suspension des protéines

Le maintien en suspension des assemblages protéiques et des matières premières a été apprécié par le suivi de la cinétique de sédimentation pendant 3 heures par une caméra. Les images prises toutes les minutes sont analysées par le logiciel et les valeurs des niveaux de gris restitués sous format numérique.

### B. La mesure de la solubilité

La solubilité des protéines dans les assemblages protéiques a été comparée avec la solubilité des protéines dans les matières premières de départ. Pour mesurer la solubilité, des centrifugations ont été réalisées à 5 000 g pendant 10 minutes à 20°C, et la concentration en protéines a été déterminée dans les surnageants.

### C. La mesure de la dispersibilité

La dispersibilité des protéines dans les assemblages protéiques a été comparée avec la dispersibilité des protéines dans les matières premières de départ. Pour mesurer la dispersibilité, des centrifugations ont été réalisées à 300 g pendant 5 minutes à 20°C, et la concentration en protéines a été déterminée dans les surnageants.

### D. Résultats

La première série de résultats concerne les protéines de lait PROMILK 852B et les protéines de pois UF, pris seules et dans l'assemblage.

Le tableau 2 présente le taux de solubilité et la dispersibilité des assemblages obtenus avec les trois stratégies de pH. Le témoin (initial) correspond au dosage de la concentration en protéines sur l'assemblage brut sans centrifugation préalable.

Le tableau 3 présente la solubilité des protéines seules, et de l'assemblage pour les trois pH d'assemblage testés.

**TABLEAU 2 Dispersibilité et solubilité des assemblages obtenus avec les trois stratégies de pH**

| | Initiale (32 mg 852B + 8mg pois UF/ml) | 300 g (dispersibilit é) | 5000 g (solubilité) |
|---|---|---|---|
| pH 7 | 100% | 100% | 100% |
| pH 5.8 | 100% | 100% | 80% |
| pH 5.8 puis remonté à 6.9 | 100% | 100% | 75% |

**TABLEAU 3 : Concentration (mg/ml) en protéines solubles dans les dispersions initiales et le mélange**

| | pH 7 | pH 5.8 | pH 5.8 à 6.9 |
|---|---|---|---|
| PROMILK 852 B (mg/ml) | 32 | 25 | 27 |
| Pois UF (mg/ml) | 4 | 1 | 3 |
| Assemblage PROMILK 852 B + UF (mg/ml) | 39 | 32 | 30 |
| Concentration obtenue/concent ration initiale * | 0, 975 | 0,80 | 0, 75 |

| | | | |
|---|---|---|---|
| * la concentration initiale est 40 mg/ml (32 mg/ml de PROMILK 852B et 8 mg/ml de pois UF) | | | |

Les résultats ci-dessus montrent que la solubilité (fraction soluble après 5000 g pendant 10 minutes) des protéines dans les assemblages pois/caséines est dépendante de la stratégie de mélange.
L'assemblage à pH 5.8 remonté à 6.9 aboutit à des concentrations en protéines finales solubles égales à l'addition des concentrations en protéines solubles dans les matières premières (protéines UF et caséines).
Quant aux assemblages à pH 7 et pH 5.8, la concentration de protéines solubles de l'assemblage est superieure à la somme des concentrations de protéines solubles dans les matières premières (PROMILK 852 B et protéines UF). Dans ce cas, la solubilité des protéines est améliorée dans ces deux assemblages.
Ceci démontre parfaitement qu'il y a un effet synergique au niveau de la solubilité des protéines dans les assemblages aux pH 5,8 et pH 7.

La seconde série de résultats concerne les caséines PROMILK 852B et les protéines de pois Nutralys® S85 M prises seules et dans l'assemblage.
Les mêmes tableaux sont présentés.

**TABLEAU 4: Dispersibilité et solubilité des assemblages obtenus avec les trois stratégies de pH**

| | Initiale (32 mg 852B + 8mg pois Nutralys/ml) | 300 g (dispersibilité) | 5000 g (solubilité) |
|---|---|---|---|
| pH 7 | 100% | 98% | 95% |
| pH 6 | 100% | 98% | 98% |
| pH 6 puis remonté à 6.9 | 100% | 98% | 98% |

**TABLEAU 5: Concentration (mg/ml) en protéines solubles dans les dispersions initiales et le mélange**

| | pH 7 | pH 5.8 | pH 5.8 à 6.9 |
|---|---|---|---|
| PROMILK 852 B (mg/ml) | 32 | 25 | 27 |
| Pois Nutralys (mg/ml) | 2 | 0 | 2 |
| Assemblage PROMILK 852 B + Nutralys (mg/ml) | 38 | 38 | 39 |
| Concentration obtenue/concentration initiale * | 0,95 | 0,95 | 0,975 |

| | | | |
|---|---|---|---|
| * la concentration initiale est 40 mg/ml (32 mg/ml de PROMILK 852B et 8 mg/ml de pois Nutralys). | | | |

Les résultats ci-dessus montrent que la solubilité (fraction soluble après 5000 g pendant 10 minutes) des protéines dans les assemblages pois/caséines est dépendante de la stratégie de mélange.
Dans les trois assemblages, une nette amélioration de la solubilité des protéines est à noter.
En effet, les concentrations de protéines solubles dans les assemblages sont plus élevées que la somme des concentrations de protéines solubles dans les matières premières (39 > 27 + 2, 38 > 25 + 0 et 38 > 32 + 2).

Ceci démontre parfaitement qu'il y a un effet synergique au niveau de la solubilité des protéines dans les trois cas.

La troisième série de résultats concerne les caséines PROMILK 852B et le floculat de protéines de pois pris seuls et dans l'assemblage.

Le floculat de protéines de pois possède un ph de 4,5. Il a donc été mélangé selon le mode opératoire décrit ci-dessous aux caséines 852B, et l'assemblage final possède un pH de 6,5.

### Stratégie d'assemblage :

- Les protéines de pois dans le floculat sont à une concentration de 16 mg/ml et pH 4,5.
- Les caséines sont à une concentration de 64mg/ml et pH 7.
- Mélange des protéines de pois aux protéines laitières 50/50 (v/v).
- pH final de l'assemblage : 6,5
- Homogénéisation par deux passages à 300 bars à température ambiante.

Les mêmes tableaux sont présentés.

**TABLEAU 6 Dispersibilité et solubilité des assemblages obtenus avec les différentes stratégies de pH**

| | Initiale (32 mg 852B + 8mg Floculat/ml) | 300 g (dispersibilité) | 5000 g (solubilité) |
|---|---|---|---|
| pH 6,5 | 100% | 100% | 100% |
| pH 7 | 100% | 100% | 100% |
| pH 5,8 | 100% | 100% | 80% |
| pH 5,8 puis remonté à 6.9 | 100% | 100% | 100% |

**TABLEAU 7: Concentration (mg/ml) en protéines solubles dans les dispersions initiales et le mélange**

| | pH 6,5 | pH 7 | pH 5.8 | pH 5.8 à 6.9 |
|---|---|---|---|---|
| PROMILK 852 B (mg/ml) | 32 | 32 | 25 | 27 |
| Floculat (mg/ml) | 0 | 2 | 0 | 3 |
| Assemblage 852 B + Floculat (mg/ml) | 40 | 40 | 32 | 40 |
| Concentration obtenue/concentration initiale* | 1 | 1 | 0, 8 | 1 |

| | | | | |
|---|---|---|---|---|
| * la concentration initiale est 40 mg/ml (32 mg/ml de PROMILK 852B et 8 mg/ml de floculat). | | | | |

Les résultats ci-dessus montrent que la solubilité (fraction soluble après 5000 g pendant 10 minutes) des protéines dans les assemblages pois/caséines est améliorée quelque soit la stratégie d'assemblage (pH 6,5, pH 7, pH 5,8 et ph 5,8 avec remontée à 6,9).

Il ressort très clairement de ce dernier exemple qu'il y a bien un effet synergique dans les assemblages réalisés entre les protéines laitières et les protéines végétales selon la présente invention.

### Exemple 5 : Aptitude technologique des assemblages protéiques obtenus

Afin d'étudier les aptitudes technologiques des assemblages protéiques, nous avons travaillé sur un ratio 80/20 (caséines/pois) afin d'atteindre une concentration protéique de 40 mg/ml dans les assemblages.

### A. Screening des conditions optimales de coagulation (concentration GDL et présure)

La Glunono-delta-lactone (GDL) est un ester cyclique d'acide D-gluconique. Ce dernier est obtenu par fermentation du glucose puis cristallisé sous forme de GDL. La GDL est un agent acidogène qui permet une acidification progressive, contrairement aux autres acides alimentaires. Elle est utilisée dans les fromages, les produits à base de viande, le tofu, les produits de cuisson.
La présure est un coagulant d'origine animale extrait du quatrième estomac (caillette) de veau et de jeunes bovins. Elle est constituée d'enzymes actives appelées chymosine. Elle est destinée à l'industrie laitière pour la coagulation du lait pour la fabrication des fromages.

Pour ce faire, nous avons travaillé sur une référence laitière PROMILK 852 B à 40 mg/ml et sur les mélanges UF + 852 B à pH 7 et 5.8 remonté à 6.9.
Pour chacune de ces fractions, les concentrations nécessaires en GDL pour atteindre un pH de 4,6 et en présure ont été déterminées avec et sans traitement thermique (70°C pendant 15 secondes pour la présure et 92°C pendant 5 minutes pour la GDL).

La concentration optimale de GDL pour atteindre un pH de 4.6 au bout de 6 heures à 23°C est de 1.9% (P/V) pour toutes les fractions protéiques.
La concentration de présure qui coagule les protéines à 33°C est de 1/400 (V/V) pour toutes les fractions protéiques.

### B. La coagulation GDL

Les cinétiques de coagulation des fractions protéiques en rhéologie ont été suivies.
La résistance de la pâte évolue continûment avant et après ces points de repères. La coagulation peut être mise en évidence par la rhéomètrie dynamique. En effet, si l'on applique à la pâte une déformation sinusoïdale suffisamment faible pour ne pas la détruire, et que l'on mesure la contrainte induite dans le matériau par la déformation, il est possible de mesurer le module élastique (G') exprimant le caractère solide du matériau et le module visqueux (G'') exprimant le caractère liquide du matériau en fonction du temps. Après la fin de la coagulation, le caillé passe d'un caractère liquide G'' > G' à un caractère solide G' > G''. Cette transition rapide est due à la coagulation des fractions protéiques.

L'évolution des modules G' et G'' en fonction du temps à une déformation de 1% et une fréquence de 1% ont été déterminés Ces suivis ont été effectués sur une géométrie plan-plan striés. L'allure des courbes de coagulation des mélanges est semblable à celle du lait mais les modules finaux sont assez différents.
L'évolution du pH a également été suivie pendant toute la coagulation (6 heures).
Une appréciation visuelle de la cohésion des gels a également été réalisée.
Le tableau 8 ci-dessous résume les valeurs des modules G' finaux et les pH de coagulation ainsi que les max de Tg delta pour les fractions traitées thermiquement.

**TABLEAU 8**

| | Sans Traitement Thermique | | Traitement Thermique à 92°C pdt 5 min | | |
|---|---|---|---|---|---|
| | G' final (Pa) | pH de gélification | G' final (Pa) | pH de gélification | Tg delta max |
| 852 B 4% (40 mg/ml de protéines) | 173 | 5,6 | 272 | 5,3 | 0,52 - pH 5,15 |
| Assemblage UF + 852 B pH 7 | 159 | 5,9 | 242 | 5,4 | 0,43 - pH 5,15 |
| Assemblage UF + 852 B pH 5.8 à 6.9 | 201 | 5,6 | 147 | 5,3 | 0,47 - pH 5,17 |
| Assemblage Nutralys + 852 B pH 7 | 200 | 5,5 | **420** | 5,4 | 0,46 - pH 5,2 |
| Assemblage Nutralys + 852 B pH 6 à 6.9 | **392** | 5,5 | **413** | 5,2 | 0,46 - pH 5,2 |
| Assemblage Floculat + 852 B pH 7 | **420** | 5,6 | **460** | 5,5 | 0,48 - pH 5,3 |
| Assemblage Floculat +852 B pH 5.8 puis remonté à 6.9 | **527** | 5,6 | 231 | 5,2 | 0,48 - pH 5,3 |

### C. Conclusions sur la coagulation à la GDL

L'assemblage Nutralys + PROMILK 852 B : Les modules G' finaux sont multipliés par un facteur 2 par rapport à ceux de 852 B 4%, en particulier sur les associations avec traitement thermique.
L'assemblage Floculat +852 B : Les modules G' finaux sont multipliés par un facteur 2 par rapport à ceux de 852 B 4%, en particulier pour les associations sans traitement thermique.

L'apport de protéines de pois (UF, Nutralys et Floculat) aux caséines (PROMILK 852 B) dans une proportion (80/20) permet d'obtenir des gels GDL avec des G' finaux supérieurs à ceux de la protéine laitière (PROMILK 852 B 4%).

### D. La coagulation à la présure

Dans l'industrie fromagère qui est basée sur la coagulation présure, la qualité d'un gel est jugée en fonction de sa texture (caractéristiques rhéologiques), ainsi que sur la récupération des protéines (le moins de perte possible dans l'exsudat).

Lors des mesures de cinétiques de coagulation à la présure, il a été démontré que les assemblages protéines de lait/protéines végétales selon la présente invention possédaient des cinétiques de coagulation intéressantes. En effet, les cinétiques de coagulation des assemblages protéines de lait/protéines végétales selon la présente invention sont comparables aux cinétiques de coagulation des protéines laitières.
L'apport de protéines de pois à des protéines de lait selon la présente invention ne perturbe donc pas les cinétiques de coagulation à la présure.

Comme le montre la figure 1, l'apport de protéines de pois sous différentes formes et selon différents procédés d'assemblage aboutit à des pertes de protéines comparables à celles obtenues avec la solution protéique à base de PROMILK 852 B, dans les conditions opératoires du laboratoire.

## Revendications

1. Procédé d'obtention d'un assemblage d'au moins une protéine végétale et d'au moins une protéine laitière, comprenant les étapes consistant à se munir d'une composition comprenant au moins une protéine végétale, à se munir d'une composition comprenant au moins une protéine laitière et à mélanger la composition comprenant au moins une protéine végétale et la composition comprenant au moins une protéine laitière, et en outre une ou plusieurs étapes, identiques ou différentes de traitement modifiant la conformation des protéines, **caractérisé en ce que** la protéine végétale appartient aux protéines de légumineuses,
et **en ce que** le procédé comprend une étape de traitement modifiant la conformation des protéines qui consiste à abaisser le pH de la composition comprenant au moins une protéine végétale à une valeur inférieure ou égale à 4, avant le mélange avec la composition comprenant au moins une protéine laitière.

2. Procédé selon la revendication 1, **caractérisé en ce que** la protéine de légumineuse est choisie dans le groupe consistant en la luzerne, le trèfle, le lupin, le pois, le haricot, la fève, la fèverole et la lentille, et leurs mélanges.

3. Procédé selon l'une quelconque des revendications 1 ou 2, **caractérisé en ce que** la composition comprenant au moins une protéine laitière est une composition comprenant au moins une caséine.

4. Procédé selon l'une quelconque des revendications 1 à 3, **caractérisé en ce qu'**il comprend plusieurs étapes de traitement modifiant la conformation des protéines, les traitements pouvant être ou non de même nature, et pouvant être appliqués à des compositions différentes ou successivement à la même composition.

5. Procédé selon la revendication 1, **caractérisé en ce qu'**il comprend, en outre, une étape de remontée du pH de la composition obtenue après mélange à une valeur comprise entre 5 et 8.

6. Procédé selon l'une quelconque des revendications 1 à 5, **caractérisé en ce qu'**il comprend une étape de traitement modifiant la conformation des protéines qui consiste en une étape d'homogénéisation de la composition obtenue après mélange.

7. Procédé selon l'une quelconque des revendications 1 à 6, **caractérisé en ce qu'**il comprend les étapes consistant à :
- se munir d'une composition aqueuse comprenant au moins une protéine végétale ;
- abaisser le pH de ladite composition à une valeur inférieure ou égale à 4 pour obtenir une composition acidifiée ;
- introduire au moins une protéine laitière dans ladite composition acidifiée pour obtenir un mélange ;
- homogénéiser le mélange obtenu ;
- remonter le pH dudit mélange homogénéisé à une valeur comprise entre 5 et 8 pour obtenir ledit assemblage.

8. Assemblage d'au moins une protéine laitière et d'au moins une protéine végétale susceptible d'être obtenu par le procédé selon l'une quelconque des revendications 1 à 7.

9. Assemblage selon la revendication 8, **caractérisé en ce qu'**il se présente sous la forme d'une composition aqueuse, d'une composition aqueuse concentrée ou d'une poudre.

10. Utilisation de l'assemblage selon l'une ou l'autre des revendications 8 ou 9, **caractérisée en ce que** l'assemblage est utilisé en tant qu'agent fonctionnel, et de préférence en tant qu'agent émulsifiant, agent moussant, agent gélifiant, agent viscosant, agent foisonnant, agent rétenteur d'eau, agent filmogène et/ou adhésif, agent ayant un pouvoir dans les réactions de Maillard, agent modifiant les propriétés sensorielles des matrices alimentaires dans lesquelles il est utilisé.

11. Utilisation de l'assemblage selon l'une ou l'autre des revendications 8 ou 9, pour la réalisation d'une formulation alimentaire choisie dans le groupe constitué par les boissons, les produits laitiers, les desserts lactés, les préparations destinées à la nutrition clinique et/ou à des individus souffrant de dénutrition, les préparations destinées à la nutrition infantile, les mélanges de poudres destinés à des produits de régime, ou pour sportifs, les produits hyperprotéinés pour la nutrition diététique ou particulière, les soupes, sauces, et aides culinaires, les confiseries, comme le chocolat par exemple et tous les produits dérivés de ce dernier, les produits à base de viande, plus particulièrement dans les secteurs des pâtes fines et des saumures, notamment dans la fabrication de jambons et des charcuteries, les produits à base de poissons, comme les produits à base de surimi, les produits céréaliers comme le pain, les pâtes, biscuits, pâtisseries, céréales et barres, les produits végétariens et plats cuisinés, les produits fermentés à base de protéines végétales, comme le tofu par exemple, les agents blanchissants tels les coffee whiteners, les produits destinés à l'alimentation animale, comme par exemple les produits destinés à l'alimentation des veaux.

12. Utilisation de l'assemblage selon l'une ou l'autre des revendications 8 ou 9, pour la fabrication d'un produit laitier choisi dans le groupe constitué par les fromages frais et affinés, les fromages tartinables, les laits fermentés, les smoothies au lait, les yaourts, les spécialités laitières et les glaces fabriquées à partir de lait.

## Patentansprüche

1. Verfahren zur Erlangung einer Anordnung aus mindestens einem Pflanzenprotein und mindestens einem Milchprotein, das die folgenden Schritte umfasst: Bereitstellen einer Zusammensetzung umfassend mindestens ein Pflanzenprotein, Bereitstellen einer Zusammensetzung umfassend mindestens ein Milchprotein und Mischen der Zusammensetzung, welche mindestens ein Pflanzenprotein umfasst und der Zusammensetzung, welche mindestens ein Milchprotein umfasst, und weiterhin ein oder mehrere identische oder verschiedene Verarbeitungsschritte, welche die Konformation der Proteine modifizieren, **dadurch gekennzeichnet, dass** das Pflanzenprotein zu den Hülsenfruchtproteinen gehört und dass das Verfahren einen Verarbeitungsschritt umfasst, welcher die Konformation der Proteine modifiziert, der darin besteht, den pH-Wert der Zusammensetzung, welche mindestens eine Pflanzenprotein umfasst, auf einen Wert kleiner oder gleich 4 abzusenken, vor dem Mischen mit der Zusammensetzung, welche mindestens ein Milchprotein umfasst.

2. Verfahren nach Anspruch 1, **dadurch gekennzeichnet, dass** das Hülsenfruchtprotein ausgewählt wird aus der Gruppe bestehend aus Luzerne, Klee, Lupine, Erbse, Bohne, Saubohne, Ackerbohne und Linse und Mischungen davon.

3. Verfahren nach einem der Ansprüche 1 oder 2, **dadurch gekennzeichnet, dass** die Zusammensetzung, welche mindestens ein Milchprotein umfasst, eine Zusammensetzung ist, die mindestens ein Kasein umfasst.

4. Verfahren nach einem der Ansprüche 1 bis 3, **dadurch gekennzeichnet, dass** es mehrere Verarbeitungsschritte umfasst, welche die Konformation der Proteine modifizieren, wobei die Verarbeitungen gleichartig oder unterschiedlich sein können und auf unterschiedliche Zusammensetzungen oder nacheinander auf die gleiche Zusammensetzung angewendet werden können.

5. Verfahren nach Anspruch 1, **dadurch gekennzeichnet, dass** es weiterhin einen Schritt der Erhöhung des pH-Werts der nach dem Mischen erhaltenen Zusammensetzung auf einen Wert zwischen 5 und 8 umfasst.

6. Verfahren nach einem der Ansprüche 1 bis 5, **dadurch gekennzeichnet, dass** es einen Verarbeitungsschritt umfasst, welcher die Konformation der Proteine modifiziert, der aus einem Homogenisierungsschritt der nach dem Mischen erhaltenen Zusammensetzung besteht.

7. Verfahren nach einem der Ansprüche 1 bis 6, **dadurch gekennzeichnet, dass** es die folgenden Schritte umfasst:
- Bereitstellen einer wässrigen Zusammensetzung umfassend mindestens ein Pflanzenprotein ;
- Senken des pH-Werts der Zusammensetzung auf einen Wert kleiner oder gleich 4 um eine angesäuerte Zusammensetzung zu erhalten ;
- Einführen mindestens eines Milchproteins in die angesäuerte Zusammensetzung um eine Mischung zu erhalten ;
- Homogenisieren der erhaltenen Mischung ;
- Erhöhen des pH-Werts der homogenisierten Mischung auf einen Wert zwischen 5 und 8 um die Anordnung zu erhalten.

8. Anordnung aus mindestens einem Milchprotein und mindestens einem Pflanzenprotein, die durch das Verfahren nach einem der Ansprüche 1 bis 7 erhalten werden kann,

9. Anordnung nach Anspruch 8, **dadurch gekennzeichnet, dass** sie in Form einer wässrigen Zusammensetzung, einer konzentrierten wässrigen Zusammensetzung oder eines Pulvers vorliegt.

10. Verwendung der Anordnung nach Anspruch 8 oder 9, **dadurch gekennzeichnet, dass** die Anordnung als funktionelle Wirkstoff verwendet wird, vorzugsweise als Emulgator, Schaummittel, Geliermittel, Viskositätsmittel, Quellendes Mittel, Wasserhaltemittel, filmbildendes Mittel und/oder Klebstoff, zur Maillard-Reaktion geeignetes Mittel, Mittel zur Modifizierung der sensorischen Eigenschaften von Nährungsmittelmatrizen, in denen es verwendet ist.

11. Verwendung der Anordnung nach Anspruch 8 oder 9, zur Herstellung einer Ernährungsformulierung ausgewählt aus der Gruppe bestehend aus Getränken, Milchprodukten, Milchnachspeisen, für die klinische Ernährung und/oder für mangelernährte Personen bestimmte Zubereitungen, für Kinderernährung bestimmte Zubereitungen, für die Diätprodukte oder für Sportler bestimmte Pulvermischungen, proteinreichen Produkten für die diätetische Ernährung oder sonderernährung, Suppen, Soßen und kulinarischen Beigaben, Süßwaren wie zum Beispiel Schokolade und jegliche Nebenprodukte davon, Fleischprodukte, vor allem im Bereich feiner Teigwaren und Salzlaken, insbesondere bei der Herstellung von Schinken und Wurstwaren, Fischprodukten wie etwa Surimiprodukten, Getreideprodukten wie etwa Brot, Teigwaren, Keksen, Gebäck, Getreide und Riegeln, vegetarischen Produkten und Fertiggerichten, fermentierten Produkten auf Basis pflanzlicher Proteine wie zum Beispiel Tofu, Bleichmitteln wie etwa Kaffeeweißer, für die Tiernahrung bestimmte Produkte wie zum Beispiel Produkte zur Kälberernährung bestimmte Produkte.

12. Verwendung der Anordnung nach Anspruch 8 oder 9, zur Herstellung eines Milchprodukts ausgewählt aus der Gruppe bestehend aus frischen und gereiften Käsen, streichfähigen Käsen, fermentierten Milch, Smoothies aus Milch, Joghurts, Milchspezialitäten und aus Milch hergestellten Eis.

## Claims

1. A process for obtaining an assembly of at least one vegetable protein and at least one dairy protein, comprising the steps consisting in obtaining a composition comprising at least one vegetable protein, in obtaining a composition comprising at least one dairy protein and in mixing the composition comprising at least one vegetable protein and the composition comprising at least one dairy protein, and in addition one or more identical or different steps of treatment which modifies the conformation of the proteins,
**characterized in that** the vegetable protein belongs to the leguminous plant proteins and **in that** said process comprises a step of treatment which modifies the conformation of the proteins consisting in lowering the pH of the composition comprising at least one vegetable protein to a value less than or equal to 4, before the mixing with the composition comprising at least one dairy protein.

2. The process as claimed in claim 1, **characterized in that** the leguminous plant protein is chosen from the group consisting of alfalfa, clover, lupin, pea, bean, broad bean, horse bean and lentil, and mixtures thereof.

3. The process as claimed in either of claims 1 or 2, **characterized in that** the composition comprising a least one dairy protein is a composition comprising at least one casein.

4. The process as claimed in any one of claims 1 to 3, **characterized in that** it comprises several steps of treatment which modifies the conformation of the proteins, it being possible for the treatments to be optionally of the same nature, and to be applied to different compositions or successively to the same composition.

5. The process as claimed in claim 1, **characterized in that** it also comprises a step of raising the pH of the composition obtained after mixing to a value between 5 and 8.

6. The process as claimed in any one of claims 1 to 5, **characterized in that** it comprises a step of treatment which modifies the conformation of the proteins consisting of a step of homogenization of the composition obtained after mixing.

7. The process as claimed in any one of claims 1 to 5, **characterized in that** it comprises the steps consisting in:
- obtaining an aqueous composition comprising at least one vegetable protein;
- lowering the pH of said composition to a value less than or equal to 4 so as to obtain an acidified composition;
- introducing at least one dairy protein into said acidified composition so as to obtain a mixture;
- homogenizing the mixture obtained;
- raising the pH of said homogenized mixture to a value between 5 and 8 so as to obtain said assembly.

8. An assembly of at least one dairy protein and at least one vegetable protein which can be obtained by means of the process as claimed in any one of claims 1 to 7.

9. The assembly as claimed in claim 8, **characterized in that** it is in the form of an aqueous composition, of a concentrated aqueous composition or of a powder.

10. The use of the assembly as claimed in either of claims 8 and 9, **characterized in that** the assembly is used as a functional agent, and preferably as an emulsifier, a foaming agent, a gelling agent, a viscosifying agent, an overrun agent, a water-retaining agent, a film-forming and/or adhesive agent, an agent which has a capacity in Maillard reactions, or an agent which modifies the sensory properties of the food matrices in which it is used.

11. The use of the assembly as claimed in either of claims 8 and 9, for the production of a food formulation chosen from the group made up of beverages, dairy products, milk desserts, preparations intended for clinical nutrition and/or for individuals suffering from undernourishment, preparations intended for infant nutrition, mixtures of powders intended for diet products, or for sportspersons, hyperproteinated products for dietetic or specific nutrition, soups, sauces and cooking aids, confectionary products, for instance chocolate and all the products derived from the latter, meat-based products, more particularly in the fine paste and brine sectors, in particular in the production of hams and cooked pork meats, fish-based products, such as surimi-based products, cereal products such as bread, pasta, cookies, pastries, cereals and bars, vegetarian products and ready meals, fermented products based on vegetable proteins, for instance tofu, whitening agents such as coffee whiteners, products intended for feeding animals, for instance products intended for feeding calves.

12. The use of the assembly as claimed in either of claims 8 and 9, for the production of a dairy product chosen from the group made up of fresh cheeses and ripened cheeses, cheese spreads, fermented milks, milk smoothies, yoghurts, specialty dairy products, and ice creams produced from milk.
